(19) Europäisches Patentamt
European Patent Office
Office européen des brevets

(11) EP 3 118 233 A1

(12) **EUROPEAN PATENT APPLICATION**
published in accordance with Art. 153(4) EPC

(43) Date of publication:
**18.01.2017 Bulletin 2017/03**

(21) Application number: **15760947.0**

(22) Date of filing: **11.03.2015**

(51) Int Cl.:
***C08G 18/38** (2006.01)* ***C08G 18/79** (2006.01)*
***G02B 1/04** (2006.01)* ***G02C 7/02** (2006.01)*
***G02C 7/12** (2006.01)*

(86) International application number:
**PCT/JP2015/057165**

(87) International publication number:
**WO 2015/137401 (17.09.2015 Gazette 2015/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **11.03.2014 JP 2014047890**
**12.09.2014 JP 2014185995**

(71) Applicant: **Mitsui Chemicals, Inc.**
**Tokyo 105-7117 (JP)**

(72) Inventors:
- **TSUKADA, Hidetaka**
  **Omuta-shi**
  **Fukuoka 836-8610 (JP)**
- **GOTO, Kenichi**
  **Sodegaura-shi**
  **Chiba 299-0265 (JP)**
- **NAKAGAWA, Toshihiko**
  **Sodegaura-shi**
  **Chiba 299-0265 (JP)**
- **YAMASAKI, Satoshi**
  **Sodegaura-shi**
  **Chiba 299-0265 (JP)**
- **KAWAGUCHI, Masaru**
  **Omuta-shi**
  **Fukuoka 836-8610 (JP)**

(74) Representative: **Wills, Andrew Jonathan et al**
**Mewburn Ellis LLP**
**City Tower**
**40 Basinghall Street**
**London EC2V 5DE (GB)**

# (54) POLYMERIZABLE COMPOSITION FOR OPTICAL MATERIAL, AND OPTICAL MATERIAL

(57) A polymerizable composition for an optical material of the present invention includes polyisocyanate (a) including aliphatic polyisocyanate (a1) and a modified aliphatic polyisocyanate (a2), an episulfide (b), and a polythiol (c).

EP 3 118 233 A1

SEQUENCE LISTING

```
<110>   Mitsui Chemicals, Inc.

<120>   POLYMERIZABLE COMPOSITION FOR OPTICAL MATERIAL, OPTICAL MATERIAL

<130>   AJW/FP7225709

<140>   EP15760947.0
<141>   2015-03-11

<150>   PCT/JP2015/057165
<151>   2015-03-11

<150>  JP2014-047890
<151>  2014-03-11

<150>  JP2014-185995
<151>  2014-09-12

<160>  4

<170>  PatentIn version 3.3

<210>  1
<211>  47
<212>  DNA
<213>  Artificial

<220>
<223>  Primer

<400>  1
aaggtaccac aaaaaggata aaacaatgaa cgttattgca atattga                   47


<210>  2
<211>  30
<212>  DNA
<213>  Artificial

<220>
<223>  Primer

<400>  2
agtctagatt attttttgct ttcttctttc                                      30


<210>  3
<211>  2177
<212>  DNA
<213>  Escherichia coli


<220>
<221>  CDS
<222>  (24)..(2171)

<400>  3
ggtaccacaa aaaggataaa aca atg aac gtt att gca ata ttg aat cac atg     53
                          Met Asn Val Ile Ala Ile Leu Asn His Met
                          1               5                   10
```

ggg gtt tat ttt aaa gaa gaa ccc atc cgt gaa ctt cat cgc gcg ctt 101
Gly Val Tyr Phe Lys Glu Glu Pro Ile Arg Glu Leu His Arg Ala Leu
15 20 25

gaa cgt ctg aac ttc cag att gtt tac ccg aac gac cgt gac gac tta 149
Glu Arg Leu Asn Phe Gln Ile Val Tyr Pro Asn Asp Arg Asp Asp Leu
30 35 40

tta aaa ctg atc gaa aac aat gcg cgt ctg tgc ggc gtt att ttt gac 197
Leu Lys Leu Ile Glu Asn Asn Ala Arg Leu Cys Gly Val Ile Phe Asp
45 50 55

tgg gat aaa tat aat ctc gag ctg tgc gaa gaa att agc aaa atg aac 245
Trp Asp Lys Tyr Asn Leu Glu Leu Cys Glu Glu Ile Ser Lys Met Asn
60 65 70

gag aac ctg ccg ttg tac gcg ttc gct aat acg tat tcc act ctc gat 293
Glu Asn Leu Pro Leu Tyr Ala Phe Ala Asn Thr Tyr Ser Thr Leu Asp
75 80 85 90

gta agc ctg aat gac ctg cgt tta cag att agc ttc ttt gaa tat gcg 341
Val Ser Leu Asn Asp Leu Arg Leu Gln Ile Ser Phe Phe Glu Tyr Ala
95 100 105

ctg ggt gct gct gaa gat att gct aat aag atc aag cag acc act gac 389
Leu Gly Ala Ala Glu Asp Ile Ala Asn Lys Ile Lys Gln Thr Thr Asp
110 115 120

gaa tat atc aac act att ctg cct ccg ctg act aaa gca ctg ttt aaa 437
Glu Tyr Ile Asn Thr Ile Leu Pro Pro Leu Thr Lys Ala Leu Phe Lys
125 130 135

tat gtt cgt gaa ggt aaa tat act ttc tgt act cct ggt cac atg ggc 485
Tyr Val Arg Glu Gly Lys Tyr Thr Phe Cys Thr Pro Gly His Met Gly
140 145 150

ggt act gca ttc cag aaa agc ccg gta ggt agc ctg ttc tat gat ttc 533
Gly Thr Ala Phe Gln Lys Ser Pro Val Gly Ser Leu Phe Tyr Asp Phe
155 160 165 170

ttt ggt ccg aat acc atg aaa tct gat att tcc att tca gta tct gaa 581
Phe Gly Pro Asn Thr Met Lys Ser Asp Ile Ser Ile Ser Val Ser Glu
175 180 185

ctg ggt tct ctg ctg gat cac agt ggt cca cac aaa gaa gca gaa cag 629
Leu Gly Ser Leu Leu Asp His Ser Gly Pro His Lys Glu Ala Glu Gln
190 195 200

tat atc gct cgc gtc ttt aac gca gac cgc agc tac atg gtg acc aac 677
Tyr Ile Ala Arg Val Phe Asn Ala Asp Arg Ser Tyr Met Val Thr Asn
205 210 215

ggt act tcc act gcg aac aaa att gtt ggt atg tac tct gct cca gca 725
Gly Thr Ser Thr Ala Asn Lys Ile Val Gly Met Tyr Ser Ala Pro Ala
220 225 230

ggc agc acc att ctg att gac cgt aac tgc cac aaa tcg ctg acc cac 773
Gly Ser Thr Ile Leu Ile Asp Arg Asn Cys His Lys Ser Leu Thr His
235 240 245 250

ctg atg atg atg agc gat gtt acg cca atc tat ttc cgc ccg acc cgt 821
Leu Met Met Met Ser Asp Val Thr Pro Ile Tyr Phe Arg Pro Thr Arg
255 260 265

```
aac gct tac ggt att ctt ggt ggt atc cca cag agt gaa ttc cag cac      869
Asn Ala Tyr Gly Ile Leu Gly Gly Ile Pro Gln Ser Glu Phe Gln His
            270                 275                 280

gct acc att gct aag cgc gtg aaa gaa aca cca aac gca acc tgg ccg      917
Ala Thr Ile Ala Lys Arg Val Lys Glu Thr Pro Asn Ala Thr Trp Pro
        285                 290                 295

gta cat gct gta att acc aac tct acc tat gat ggt ctg ctg tac aac      965
Val His Ala Val Ile Thr Asn Ser Thr Tyr Asp Gly Leu Leu Tyr Asn
    300                 305                 310

acc gac ttc atc aag aaa aca ctg gat gtg aaa tcc atc cac ttt gac     1013
Thr Asp Phe Ile Lys Lys Thr Leu Asp Val Lys Ser Ile His Phe Asp
315                 320                 325                 330

tcc gcg tgg gtg cct tac acc aac ttc tca ccg att tac gaa ggt aaa     1061
Ser Ala Trp Val Pro Tyr Thr Asn Phe Ser Pro Ile Tyr Glu Gly Lys
                335                 340                 345

tgc ggt atg agc ggt ggc cgt gta gaa ggg aaa gtg att tac gaa acc     1109
Cys Gly Met Ser Gly Gly Arg Val Glu Gly Lys Val Ile Tyr Glu Thr
            350                 355                 360

cag tcc act cac aaa ctg ctg gcg gcg ttc tct cag gct tcc atg atc     1157
Gln Ser Thr His Lys Leu Leu Ala Ala Phe Ser Gln Ala Ser Met Ile
        365                 370                 375

cac gtt aaa ggt gac gta aac gaa gaa acc ttt aac gaa gcc tac atg     1205
His Val Lys Gly Asp Val Asn Glu Glu Thr Phe Asn Glu Ala Tyr Met
    380                 385                 390

atg cac acc acc act tct ccg cac tac ggt atc gtg gcg tcc act gaa     1253
Met His Thr Thr Thr Ser Pro His Tyr Gly Ile Val Ala Ser Thr Glu
395                 400                 405                 410

acc gct gcg gcg atg atg aaa ggc aat gca ggt aag cgt ctg atc aac     1301
Thr Ala Ala Ala Met Met Lys Gly Asn Ala Gly Lys Arg Leu Ile Asn
                415                 420                 425

ggt tct att gaa cgt gcg atc aaa ttc cgt aaa gag atc aaa cgt ctg     1349
Gly Ser Ile Glu Arg Ala Ile Lys Phe Arg Lys Glu Ile Lys Arg Leu
            430                 435                 440

aga acg gaa tct gat ggc tgg ttc ttt gat gta tgg cag ccg gat cat     1397
Arg Thr Glu Ser Asp Gly Trp Phe Phe Asp Val Trp Gln Pro Asp His
        445                 450                 455

atc gat acg act gaa tgc tgg ccg ctg cgt tct gac agc acc tgg cac     1445
Ile Asp Thr Thr Glu Cys Trp Pro Leu Arg Ser Asp Ser Thr Trp His
    460                 465                 470

ggc ttc aaa aac atc gat aac gag cac atg tat ctt gac ccg atc aaa     1493
Gly Phe Lys Asn Ile Asp Asn Glu His Met Tyr Leu Asp Pro Ile Lys
475                 480                 485                 490

gtc acc ctg ctg act ccg ggg atg gaa aaa gac ggc acc atg agc gac     1541
Val Thr Leu Leu Thr Pro Gly Met Glu Lys Asp Gly Thr Met Ser Asp
                495                 500                 505

ttt ggt att ccg gcc agc atc gtg gcg aaa tac ctc gac gaa cat ggc     1589
Phe Gly Ile Pro Ala Ser Ile Val Ala Lys Tyr Leu Asp Glu His Gly
```

```
            510                 515                 520

atc gtt gtt gag aaa acc ggt ccg tat aac ctg ctg ttc ctg ttc agc      1637
Ile Val Val Glu Lys Thr Gly Pro Tyr Asn Leu Leu Phe Leu Phe Ser
        525                 530                 535

atc ggt atc gat aag acc aaa gca ctg agc ctg ctg cgt gct ctg act      1685
Ile Gly Ile Asp Lys Thr Lys Ala Leu Ser Leu Leu Arg Ala Leu Thr
    540                 545                 550

gac ttt aaa cgt gcg ttc gac ctg aac ctg cgt gtg aaa aac atg ctg      1733
Asp Phe Lys Arg Ala Phe Asp Leu Asn Leu Arg Val Lys Asn Met Leu
555                 560                 565                 570

ccg tct ctg tat cgt gaa gat cct gaa ttc tat gaa aac atg cgt att      1781
Pro Ser Leu Tyr Arg Glu Asp Pro Glu Phe Tyr Glu Asn Met Arg Ile
                575                 580                 585

cag gaa ctg gct cag aat atc cac aaa ctg att gtt cac cac aat ctg      1829
Gln Glu Leu Ala Gln Asn Ile His Lys Leu Ile Val His His Asn Leu
            590                 595                 600

ccg gat ctg atg tat cgc gca ttt gaa gtg ctg ccg acg atg gta atg      1877
Pro Asp Leu Met Tyr Arg Ala Phe Glu Val Leu Pro Thr Met Val Met
        605                 610                 615

act ccg tat gct gca ttc cag aaa gag ctg cac ggt atg acc gaa gaa      1925
Thr Pro Tyr Ala Ala Phe Gln Lys Glu Leu His Gly Met Thr Glu Glu
    620                 625                 630

gtt tac ctc gac gaa atg gta ggt cgt att aac gcc aat atg atc ctt      1973
Val Tyr Leu Asp Glu Met Val Gly Arg Ile Asn Ala Asn Met Ile Leu
635                 640                 645                 650

ccg tac ccg ccg gga gtt cct ctg gta atg ccg ggt gaa atg atc acc      2021
Pro Tyr Pro Pro Gly Val Pro Leu Val Met Pro Gly Glu Met Ile Thr
                655                 660                 665

gaa gaa agc cgt ccg gtt ctg gag ttc ctg cag atg ctg tgt gaa atc      2069
Glu Glu Ser Arg Pro Val Leu Glu Phe Leu Gln Met Leu Cys Glu Ile
            670                 675                 680

ggc gct cac tat ccg ggc ttt gaa acc gat att cac ggt gca tac cgt      2117
Gly Ala His Tyr Pro Gly Phe Glu Thr Asp Ile His Gly Ala Tyr Arg
        685                 690                 695

cag gct gat ggc cgc tat acc gtt aag gta ttg aaa gaa gaa agc aaa      2165
Gln Ala Asp Gly Arg Tyr Thr Val Lys Val Leu Lys Glu Glu Ser Lys
    700                 705                 710

aaa taa tctaga                                                       2177
Lys
715


<210>  4
<211>  715
<212>  PRT
<213>  Escherichia coli

<400>  4

Met Asn Val Ile Ala Ile Leu Asn His Met Gly Val Tyr Phe Lys Glu
```

```
1               5                   10                  15

Glu Pro Ile Arg Glu Leu His Arg Ala Leu Glu Arg Leu Asn Phe Gln
            20                  25                  30

Ile Val Tyr Pro Asn Asp Arg Asp Asp Leu Leu Lys Leu Ile Glu Asn
        35                  40                  45

Asn Ala Arg Leu Cys Gly Val Ile Phe Asp Trp Asp Lys Tyr Asn Leu
    50                  55                  60

Glu Leu Cys Glu Glu Ile Ser Lys Met Asn Glu Asn Leu Pro Leu Tyr
65                  70                  75                  80

Ala Phe Ala Asn Thr Tyr Ser Thr Leu Asp Val Ser Leu Asn Asp Leu
                85                  90                  95

Arg Leu Gln Ile Ser Phe Phe Glu Tyr Ala Leu Gly Ala Ala Glu Asp
            100                 105                 110

Ile Ala Asn Lys Ile Lys Gln Thr Thr Asp Glu Tyr Ile Asn Thr Ile
        115                 120                 125

Leu Pro Pro Leu Thr Lys Ala Leu Phe Lys Tyr Val Arg Glu Gly Lys
    130                 135                 140

Tyr Thr Phe Cys Thr Pro Gly His Met Gly Gly Thr Ala Phe Gln Lys
145                 150                 155                 160

Ser Pro Val Gly Ser Leu Phe Tyr Asp Phe Phe Gly Pro Asn Thr Met
                165                 170                 175

Lys Ser Asp Ile Ser Ile Ser Val Ser Glu Leu Gly Ser Leu Leu Asp
            180                 185                 190

His Ser Gly Pro His Lys Glu Ala Glu Gln Tyr Ile Ala Arg Val Phe
        195                 200                 205

Asn Ala Asp Arg Ser Tyr Met Val Thr Asn Gly Thr Ser Thr Ala Asn
    210                 215                 220

Lys Ile Val Gly Met Tyr Ser Ala Pro Ala Gly Ser Thr Ile Leu Ile
225                 230                 235                 240

Asp Arg Asn Cys His Lys Ser Leu Thr His Leu Met Met Met Ser Asp
                245                 250                 255
```

```
Val Thr Pro Ile Tyr Phe Arg Pro Thr Arg Asn Ala Tyr Gly Ile Leu
            260                 265                 270

Gly Gly Ile Pro Gln Ser Glu Phe Gln His Ala Thr Ile Ala Lys Arg
        275                 280                 285

Val Lys Glu Thr Pro Asn Ala Thr Trp Pro Val His Ala Val Ile Thr
    290                 295                 300

Asn Ser Thr Tyr Asp Gly Leu Leu Tyr Asn Thr Asp Phe Ile Lys Lys
305                 310                 315                 320

Thr Leu Asp Val Lys Ser Ile His Phe Asp Ser Ala Trp Val Pro Tyr
                325                 330                 335

Thr Asn Phe Ser Pro Ile Tyr Glu Gly Lys Cys Gly Met Ser Gly Gly
            340                 345                 350

Arg Val Glu Gly Lys Val Ile Tyr Glu Thr Gln Ser Thr His Lys Leu
        355                 360                 365

Leu Ala Ala Phe Ser Gln Ala Ser Met Ile His Val Lys Gly Asp Val
    370                 375                 380

Asn Glu Glu Thr Phe Asn Glu Ala Tyr Met Met His Thr Thr Thr Ser
385                 390                 395                 400

Pro His Tyr Gly Ile Val Ala Ser Thr Glu Thr Ala Ala Ala Met Met
                405                 410                 415

Lys Gly Asn Ala Gly Lys Arg Leu Ile Asn Gly Ser Ile Glu Arg Ala
            420                 425                 430

Ile Lys Phe Arg Lys Glu Ile Lys Arg Leu Arg Thr Glu Ser Asp Gly
        435                 440                 445

Trp Phe Phe Asp Val Trp Gln Pro Asp His Ile Asp Thr Thr Glu Cys
    450                 455                 460

Trp Pro Leu Arg Ser Asp Ser Thr Trp His Gly Phe Lys Asn Ile Asp
465                 470                 475                 480

Asn Glu His Met Tyr Leu Asp Pro Ile Lys Val Thr Leu Leu Thr Pro
                485                 490                 495

Gly Met Glu Lys Asp Gly Thr Met Ser Asp Phe Gly Ile Pro Ala Ser
            500                 505                 510
```

```
Ile Val Ala Lys Tyr Leu Asp Glu His Gly Ile Val Val Glu Lys Thr
        515                 520                 525

Gly Pro Tyr Asn Leu Leu Phe Leu Phe Ser Ile Gly Ile Asp Lys Thr
    530                 535                 540

Lys Ala Leu Ser Leu Leu Arg Ala Leu Thr Asp Phe Lys Arg Ala Phe
545                 550                 555                 560

Asp Leu Asn Leu Arg Val Lys Asn Met Leu Pro Ser Leu Tyr Arg Glu
                565                 570                 575

Asp Pro Glu Phe Tyr Glu Asn Met Arg Ile Gln Glu Leu Ala Gln Asn
            580                 585                 590

Ile His Lys Leu Ile Val His His Asn Leu Pro Asp Leu Met Tyr Arg
        595                 600                 605

Ala Phe Glu Val Leu Pro Thr Met Val Met Thr Pro Tyr Ala Ala Phe
    610                 615                 620

Gln Lys Glu Leu His Gly Met Thr Glu Glu Val Tyr Leu Asp Glu Met
625                 630                 635                 640

Val Gly Arg Ile Asn Ala Asn Met Ile Leu Pro Tyr Pro Pro Gly Val
                645                 650                 655

Pro Leu Val Met Pro Gly Glu Met Ile Thr Glu Glu Ser Arg Pro Val
            660                 665                 670

Leu Glu Phe Leu Gln Met Leu Cys Glu Ile Gly Ala His Tyr Pro Gly
        675                 680                 685

Phe Glu Thr Asp Ile His Gly Ala Tyr Arg Gln Ala Asp Gly Arg Tyr
    690                 695                 700

Thr Val Lys Val Leu Lys Glu Glu Ser Lys Lys
705                 710                 715
```

**Description**

TECHNICAL FIELD

The present invention relates to a polymerizable composition for an optical material, an optical material obtained using the same, and a plastic lens.

BACKGROUND ART

Compared with inorganic lenses, plastic lenses have a low weight, are not easily cracked, and can be dyed, and thus, recently, have rapidly become widespread in optical elements such as spectacle lenses and camera lenses.

For resins for plastic lenses, there has been a demand for additional improvement in performance, and there has been a demand for an increase in the refractive index, an increase in the Abbe number, a decrease in the specific weight, an improvement of the heat resistance, and the like. Thus far, a variety of resin materials for lenses have been developed and put into use.

Among them, optical materials made of sulfide-based resins have a high refractive index and a high Abbe number and are being studied as ultrahigh refractive index materials having a refractive index of higher than or equal to 1.60. Sulfide-based resins are obtained by polymerizing a polymerizable composition including an episulfide compound (Patent Documents 1 to 4). In recent years, a variety of studies also have been carried out in order to improve the quality of episulfide compounds and resins for optical materials obtained from the episulfide compounds (Patent Documents 5 and 6).

Meanwhile, for plastic materials for which fossil resources are used, there has been a concern regarding the influences on the global environment such as resource depletion or the emission of carbon dioxide, and, in the development of materials as well, studies have been carried out regarding the utilization of biomass resources such as plant-derived materials.

Regarding epichlorohydrin as well which is a raw material of episulfide compounds, a manufacturing route in which epichlorohydrin is synthesized by means of chlorination or epoxidation of glycerin originating from natural products has been developed instead of a manufacturing route in which epichlorohydrin is synthesized from a fossil raw material (propylene, allyl alcohol, or the like) of the related art and the application of the manufacturing route to epoxy resins and the like is being studied (Patent Document 7). However, no studies are being carried out regarding optical material use.

As plastic lens materials obtained using plant-derived materials, a spectacle lens which includes a polycarbonate resin having a constituent unit derived from isosorbide as a main component and is obtained by means of injection molding is proposed (Patent Document 8). This enables the obtainment of optical lenses having high heat resistance, high strength, and low distortion which are manufactured from plant-derived materials, but there is room for improvement in optical properties such as the refractive index.

Furthermore, in Patent Document 9, a resin for which plant-derived materials are used is proposed, but this resin is not for use as an ultrahigh refractive index material having a refractive index of higher than or equal to 1.60, and any optical properties and the like of the obtained resin are not exhibited.

RELATED DOCUMENT

PATENT DOCUMENT

[Patent Document 1] Japanese Unexamined Patent Publication No. 2002-194083
[Patent Document 2] Japanese Unexamined Patent Publication No. 2000-256435
[Patent Document 3] Japanese Unexamined Patent Publication No. 2001-163874
[Patent Document 4] Pamphlet of International Publication No. WO2013/115212
[Patent Document 5] Pamphlet of International Publication No. WO2013/157490
[Patent Document 6] Japanese Unexamined Patent Publication No. 2013-142073
[Patent Document 7] PCT Japanese Translation Patent Publication No. 2013-541531
[Patent Document 8] Japanese Unexamined Patent Publication No. 2010-190919
[Patent Document 9] Japanese Unexamined Patent Publication No. 2011-225863


SUMMARY OF THE INVENTION

An object of the present invention is to provide a polymerizable composition for an optical material which has

an excellent balance among transparency, heat resistance, and the like and can be used to obtain ultrahigh refractive index plastic lens materials having a refractive index of higher than or equal to 1.60 and an optical material that can be obtained from the same.

As a result of intensive studies, the present inventors found that a certain composition for which an episulfide compound is used enables the provision of optical materials such as ultrahigh refractive index plastic lenses which have an excellent balance among transparency, refractive index, heat resistance, and the like and have a refractive index of higher than 1.60 and completed the present invention.

That is, the present invention can be described as below.

[1] A polymerizable composition for an optical material including: polyisocyanate (a) including aliphatic polyisocyanate (a1) and a modified aliphatic polyisocyanate (a2) ; an episulfide (b) ; and a polythiol (c).

[2] The polymerizable composition for an optical material according to [1], in which the modified aliphatic polyisocyanate (a2) is an isocyanurate of the aliphatic polyisocyanate.

[3] The polymerizable composition for an optical material according to [1] or [2], in which the aliphatic polyisocyanate (a1) and/or the aliphatic polyisocyanate in the modified aliphatic polyisocyanate (a2) are obtained from plant-derived materials.

[4] The polymerizable composition for an optical material according to any one of [1] to [3], in which the episulfide (b) includes an episulfide represented by General Formula (1) below:

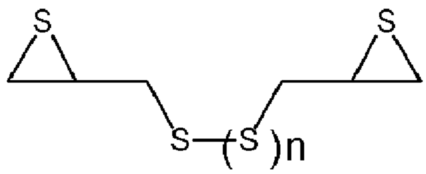

(1)

in the formula, n represents 0 or 1.

[5] The polymerizable composition for an optical material according to any one of [1] to [4], in which the episulfide (b) includes an episulfide obtained from plant-derived materials.

[6] The polymerizable composition for an optical material according to any one of [1] to [5], in which the aliphatic polyisocyanate (a1) and/or the aliphatic polyisocyanate in the modified aliphatic polyisocyanate (a2) are at least one selected from 1,5-pentamethylene diisocyanate, 1,6-hexamethylene diisocyanate, 1,7-heptamethylene diisocyanate, lysine diisocyanate, lysine triisocyanate, dimer acid diisocyanate, octamethylene diisocyanate, and decamethylene diisocyanate.

[7] The polymerizable composition for an optical material according to any one of [1] to [6], in which the polythiol (c) is at least one selected from 4-mercaptomethyl-1,8-dimercapto-3,6-dithiaoctane, 4,8 or 4,7 or 5,7-dimercaptomethyl-1,11-dimercapto-3,6,9-trithiaundecane, pentaerythritol tetrakis mercaptoacetate, pentaerythritol tetrakis mercaptopropionate, 2,5-bis(mercaptomethyl)-1,4-dithiane, bis(mercaptoethyl)sulfide, 1,1,3,3-tetrakis(mercaptomethylthio)propane, 4,6-bis(mercaptomethylthio)-1,3-dithiane, 2-(2,2-bis(mercaptomethylthio) ethyl)-1,3-dithietane, 1,1,2,2-tetrakis(mercaptomethylthio) ethane, 3-mercaptomethyl-1,5-dimercapto-2,4-dithiapentane, tris(mercaptomethylthio) methane, and ethylene glycol bis(3-mercaptopropionate).

[8] The polymerizable composition for an optical material according to any one of [1] to [7], in which the polythiol (c) is a polythiol obtained from plant-derived materials, and is selected from 4-mercaptomethyl-1,8-dimercapto-3,6-dithiaoctane and 4,8 or 4,7 or 5,7-dimercaptomethyl-1,11-dimercapto-3,6,9-trithiaundecane.

[9] A molding containing a resin obtained by polymerizing and curing the polymerizable composition for an optical material according to any one of [1] to [8].

[10] The molding according to [9] in which the resin has a degree of biomass of greater than or equal to 25%.

[11] The molding according to [9] or [10], in which the molding has a refractive index of higher than or equal to 1.60.

[12] The molding according to any one of [9] to [11], in which the molding has a glass transition temperature (Tg) of higher than or equal to 60°C.

[13] An optical material formed of the molding according to any one of [9] to [12].

[14] A plastic spectacle lens formed of the molding according to any one of [9] to [12].

[15] A plastic polarizing lens, comprising: a polarizing film; and a layer formed of the molding according to any one of [9] to [12] in which wherein the layer is laminated on at least one surface of the polarizing film.

[16] A method for manufacturing a plastic spectacle lens, comprising: a step of injecting the polymerizable composition for an optical material according to any one of [1] to [8] into a mold for lens casting; anda step of polymerizing and curing the polymerizable composition for an optical material.

[17] A method for manufacturing a plastic polarizing lens, comprising: a step of fixing a polarizing film into a lens casting mold in a state in which at least one surface of the polarizing film is separated from a mold; a step of injecting the polymerizable composition for an optical material according to any one of [1] to [8] into a gap between the polarizing film and the mold; and a step of laminating a layer formed of a polythiourethane resin on at least one surface of the polarizing film by polymerization and curing the polymerizable composition for an optical material.

According to the polymerizable composition for an optical material of the present invention, it is possible to obtain an optical material such as an ultrahigh refractive index plastic lens which has an excellent balance among transparency, refractive index, heat resistance, dyeing properties, and the like and has a refractive index of higher than or equal to 1.60.

Furthermore, when a compound obtained from plant-derived materials is used, it is possible to provide an optical material such as a plastic lens having a degree of biomass of higher than or equal to 25%.

DESCRIPTION OF EMBODIMENTS

The present invention will be described on the basis of an embodiment.

A polymerizable composition for an optical material of the present embodiment includes polyisocyanate (a) including aliphatic polyisocyanate (a1) and a modified aliphatic polyisocyanate (a2), an episulfide (b), and a polythiol (c).

First, the respective components will be described.

(Polyisocyanate (a))

The polyisocyanate (a) includes aliphatic polyisocyanate (a1) and a modified aliphatic polyisocyanate (a2).

Examples of the aliphatic polyisocyanate (a1) and the aliphatic polyisocyanate in the modified aliphatic polyisocyanate (a2) include trimethylene diisocyanate, tetramethylene diisocyanate, 1,5-pentamethylene diisocyanate, 1,6-hexamethylene diisocyanate, 1,7-heptamethylene diisocyanate, 2,2,4-trimethylhexamethylene diisocyanate, 2,4,4-trimethylhexamethylene diisocyanate, lysine diisocyanate, lysine diisocyanatomethyl ester, lysine triisocyanate, m-xylylene diisocyanate, $\alpha,\alpha,\alpha',\alpha'$-tetramethylxylylene diisocyanate, bis(isocyanatomethyl) naphthalene, mesitylene triisocyanate, bis(isocyanatomethyl)sulfide, bis(isocyanatoethyl)sulfide, bis(isocyanatomethyl)disulfide, bis(isocyanatoethyl)disulfide, bis(isocyanatomethylthio) methane, bis(isocyanatoethylthio) methane, bis(isocyanatoethylthio) ethane, bis(isocyanatomethylthio) ethane, dimer acid diisocyanate, octamethylene diisocyanate, decamethylene diisocyanate, and the like. From the viewpoint of the improvement of transparency, the adjustment of the refractive index, and the handling properties (viscosity) of compositions to be obtained, preferred examples thereof include 1,5-pentamethylene diisocyanate, 1,6-hexamethylene diisocyanate, 1,7-heptamethylene diisocyanate, lysine diisocyanate, lysine triisocyanate, dimer acid diisocyanate, octamethylene diisocyanate, decamethylene diisocyanate, and the like, and the aliphatic polyisocyanates may be used alone, or two or more may be used in combination.

Examples of the modified aliphatic polyisocyanate (a2) include a multimeric, a biuret modified, an allophanate modified, an oxadiazinetrione modified, a polyol modified aliphatic polyisocyanate, and the like.

Examples of the multimers of the aliphatic polyisocyanate include multimers such as dimers such as uretdione, urethoimine, and carbodiimide, and trimers and higher such as an isocyanurate and iminooxadiazinedione.

An isocyanurate as the aliphatic polyisocyanate can be obtained by performing a reaction of aliphatic polyisocyanate in the presence of a well-known isocyanurate-forming catalyst.

The biuret modified aliphatic polyisocyanate can be obtained by reacting aliphatic polyisocyanate and, for example, water, a tertiary alcohol (for example, t-butyl alcohol), a secondary amine (for example, dimethylamine or diethylamine), or the like, and then performing an additional reaction in the presence of a well-known biuret-forming catalyst.

The allophanate modified aliphatic polyisocyanate can be obtained by reacting aliphatic polyisocyanate and a monoalcohol (a monovalent alcohol, for example, C1 to C10 alcohol), and then performing an additional reaction in the presence of a well-known allophanate-forming catalyst.

The oxadiazinetrione modified aliphatic polyisocyanate can be obtained from a reaction between aliphatic polyisocyanate and carbon dioxide.

The iminooxadiazinedione modified aliphatic polyisocyanate can be obtained by further reacting aliphatic polyisocyanate in the presence of a well-known catalyst for forming imino-oxadiazinedione.

The polyol modified aliphatic polyisocyanate can be obtained from a reaction between aliphatic polyisocyanate and an alcohol. Alcohols that are used to obtain the modified aliphatic polyisocyanate are tertiary alcohols, monoalcohols, and polyvalent alcohols, preferred examples thereof include trivalent alcohols such as glycerin and trimethylolpropane, and the alcohols are more preferably plant-derived compounds.

The modified aliphatic polyisocyanate (a2) may be used alone, or two or more may be used in combination.

Among the modified aliphatic polyisocyanate (a2), multimers of the aliphatic polyisocyanate are preferred, an isocyanurate of the aliphatic polyisocyanate is more preferred, and a mononuclear isocyanurate (trimer) of the aliphatic polyisocyanate is particularly preferred.

In the present embodiment, the aliphatic polyisocyanate (a1) in the polyisocyanate (a) may be identical or different from aliphatic polyisocyanate used to obtain the modified aliphatic polyisocyanate (a2), and at least one kind of aliphatic polyisocyanate can be included.

As the polyisocyanate (a), it is possible to use a reaction mixture obtained when the modified aliphatic polyisocyanate (a2) is prepared. In this case, the reaction mixture includes the aliphatic polyisocyanate (a1) and the modified aliphatic polyisocyanate (a2). That is, in the present embodiment, the polyisocyanate (a) can also be represented as the polyisocyanate composition (a). In addition, the polyisocyanate (a) can also be obtained by adding at least one kind of the aliphatic polyisocyanate (a1) that is identical to or different from the aliphatic polyisocyanate used to prepare the modified aliphatic polyisocyanate to the prepared modified aliphatic polyisocyanate.

When the polyisocyanate (a) includes the modified aliphatic polyisocyanate (a2) in the form of a mononuclear aliphatic polyisocyanate (trimer) within a range of 0% by weight to 80% by weight, preferably within a range of 5% by weight to 75% by weight, and more preferably within a range of 10% by weight to 70% by weight of the polyisocyanate (a), it is possible to provide a molding formed of an optical material resin having superior properties such as transparency, refractive index, and heat resistance.

In the present embodiment, from the viewpoint of obtaining optical resins having a high degree of biomass, the aliphatic polyisocyanate (a1) or the aliphatic polyisocyanate in the modified aliphatic polyisocyanate (a2) is preferably aliphatic polyisocyanate obtained from plant-derived materials, and the aliphatic polyisocyanate (a1) and the aliphatic polyisocyanate in the modified aliphatic polyisocyanate (a2) are more preferably aliphatic polyisocyanate obtained from plant-derived materials.

As the plant-derived aliphatic polyisocyanate, it is possible to use aliphatic polyisocyanate having 5 to 18 carbon atoms, and specific examples thereof include 1,5-pentamethylene diisocyanate, 1,6-hexamethylene diisocyanate, 1,7-heptamethylene diisocyanate, lysine diisocyanate, lysine triisocyanate, dimer acid diisocyanate, octamethylene diisocyanate, decamethylene diisocyanate, and the like, and the plant-derived aliphatic polyisocyanates may be used alone, or two or more may be used in combination.

In the present embodiment, 1,5-pentamethylene diisocyanate is preferably used.

The plant-derived aliphatic polyisocyanate can be obtained by, for example, converting plant-derived divalent carboxylic acid to a terminal amino group by means of the formation of an acid amide and reduction and, furthermore, reacting the terminal amino group with phosgene so as to convert the amino group to an isocyanate group.

In addition, the plant-derived aliphatic polyisocyanate can also be obtained by using an amino acid as a plant-derived material and converting an amino group thereof to an isocyanate group.

For example, pentamethylene diisocyanate can be obtained by decarboxylating a carboxyl group of lysine and then converting an amino group to an isocyanate group. Examples of the pentamethylene diisocyanate include 1,5-pentamethylene diisocyanate.

When aliphatic polyisocyanate obtained from plant-derived materials is used as the aliphatic polyisocyanate (a1) and/or the aliphatic polyisocyanate in the modified aliphatic polyisocyanate (a2), it is possible to provide a molding formed of a resin for an optical material having a high degree of biomass of higher than or equal to 25% and having excellent properties such as transparency, refractive index, and heat resistance.

In the polymerizable composition for an optical material of the present embodiment, it is also possible to further use at least one selected from alicyclic polyisocyanate, aromatic polyisocyanate, and heterocyclic polyisocyanate in addition to the polyisocyanate (a).

Examples of the alicyclic polyisocyanate include 3-isocyanatomethyl-3,5,5-trimethyl cyclohexyl isocyanate, isophorone diisocyanate, 4,4'-, 2,4'-, or 2,2'-dicyclohexylmethane diisocyanate and mixtures thereof, 1,4- or 1,3-bis(isocyanatomethyl) cyclohexane and mixtures thereof, 1,4- or 1,3-bis(isocyanatoethyl) cyclohexane and mixtures thereof, bis(isocyanatomethyl)-bicyclo[2.2.1] heptane, 1,3-cyclopentane diisocyanate, 1,4- or 1,3-cyclohexane diisocyanate and mixtures thereof, methyl-2,4-cyclohexane diisocyanate, methyl-2,6-cyclohexane diisocyanate, 3,8-bis(isocyanatomethyl) tricyclodecane, 3,9-bis(isocyanatomethyl) tricyclodecane, 4,8-bis(isocyanatomethyl) tricyclodecane, 4,9-bis(isocyanatomethyl) tricyclodecane, and the like.

Meanwhile, bis(isocyanatomethyl)-bicyclo[2.2.1] heptane is a mixture of isomers of 2,5-bis(isocyanatomethyl)-bicyclo[2.2.1] heptane and 2,6-bis(isocyanatomethyl)-bicyclo[2.2.1] heptane. In the present embodiment, the compound formed of a mixture of these isomers can be used as a single kind of compound. The alicyclic polyisocyanates may be used alone, or two or more may be used in combination.

Examples of the aromatic polyisocyanate include tolylene diisocyanate, 4,4'-diphenylmethane diisocyanate, 2,4'-diphenylmethane diisocyanate, phenylene diisocyanate, and the like. The aromatic polyisocyanates may be used alone, or two or more may be used in combination.

Examples of tolylene diisocyanate include 2,4-tolylene diisocyanate, 2,6-tolylene diisocyanate, and mixtures

of 2, 4-tolylene diisocyanate and 2, 6-tolylene diisocyanate, and, in the present embodiment, 2,4-tolylene diisocyanate is preferably included. Specifically, 2,4-tolylene diisocyanate can be used singly, or 2,4-tolylene diisocyanate and 2,6-tolylene diisocyanate can be used in the form of mixture, and in a case in which the mixture is used, the mixing ratio between 2,4-tolylene diisocyanate and 2,6-tolylene diisocyanate is more preferably within a range of 75:25 to 85:15.

Examples of the heterocyclic polyisocyanate include 2,5-diisocyanato thiophene, 2,5-bis(isocyanatomethyl) thiophene, 2,5-diisocyanato tetrahydrothiophene, 2,5-bis(isocyanatomethyl) tetrahydrothiophene, 3,4-bis(isocyanatomethyl) tetrahydrothiophene, 2,5-diisocyanato-1,4-dithiane, 2,5-bis(isocyanatomethyl)-1,4-dithiane, 4,5-diisocyanato-1,3-dithiolane, 4,5-bis(isocyanatomethyl)-1,3-dithiolane, and the like. The heterocyclic polyisocyanates may be used alone, or two or more may be used in combination.

It is preferable that the alicyclic polyisocyanate, the aromatic polyisocyanate, the heterocyclic polyisocyanate, or the aliphatic polyisocyanate which may be used in combination is the plant-derived material, and a compound which is not obtained from the plant-derived material can be used. As it is used, the compound is may be used such that the degree of biomass of the polythiourethane resin is in a predetermined range (greater than or equal to 25%).

[Episulfide (b)]

In the present embodiment, examples of the episulfide (b) include epithioethylthio compounds such as bis(1,2-epithioethyl)sulfide, bis(1,2-epithioethyl)disulfide, bis(epithioethylthio) methane, bis(epithioethylthio) benzene, bis[4-(epithioethylthio) phenyl] sulfide, and bis[4-(epithioethylthio) phenyl] methane;
chain aliphatic 2,3-epithiopropylthio compounds such as compounds having two episulfide groups in the molecule represented by General Formula (1) below:

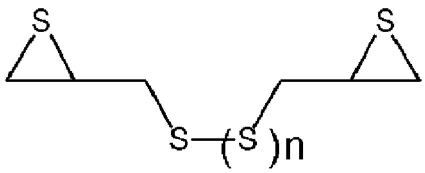

(1)

wherein in the formula (1), n represents 0 or 1; bis(2,3-epithiopropylthio) methane, 1,2-bis(2,3-epithiopropylthio) ethane, 1,2-bis(2,3-epithiopropylthio) propane, 1,3-bis(2,3-epithiopropylthio) propane, 1,3-bis(2,3-epithiopropylthio)-2-methylpropane, 1,4-bis(2,3-epithiopropylthio) butane, 1,4-bis(2,3-epithiopropylthio)-2-methylbutane, 1,3-bis(2,3-epithiopropylthio) butane, 1,5-bis(2,3-epithiopropylthio) pentane, 1,5-bis(2,3-epithiopropylthio)-2-methylpentane, 1,5-bis(2,3-epithiopropylthio)-3-thiapentane, 1,6-bis(2,3-epithiopropylthio) hexane, 1,6-bis(2,3-epithiopropylthio)-2-methylhexane, 3,8-bis(2,3-epithiopropylthio)-3,6-dithiaoctane, 1,2,3-tris(2,3-epithiopropylthio) propane, 2,2-bis(2,3-epithiopropylthio)-1,3-bis(2,3-epithiopropylthiometh yl) propane, 2,2-bis(2,3-epithiopropylthiomethyl)-1-(2,3-epithiopropylthio) butane, 1,5-bis(2,3-epithiopropylthio)-2-(2,3-epithiopropylthiomethyl)-3 -thiapentane, 1,5-bis(2,3-epithiopropylthio)-2,4-bis(2,3-epithiopropylthiometh yl)-3-thiapentane, 1-(2,3-epithiopropylthio)-2,2-bis(2,3-epithiopropylthiomethyl)-4 -thiahexane, 1,5,6-tris(2,3-epithiopropylthio)-4-(2,3-epithiopropylthiomethyl )-3-thiahexane, 1,8-bis(2,3-epithiopropylthio)-4-(2,3-epithiopropylthiomethyl)-3 ,6-dithiaoctane, 1,8-bis(2,3-epithiopropylthio)-4,5-bis(2,3-epithiopropylthiometh yl)-3,6-dithiaoctane, 1,8-bis(2,3-epithiopropylthio)-4,4-bis(2,3-epithiopropylthiometh yl)-3,6-dithiaoctane, 1,8-bis(2,3-epithiopropylthio)-2,5-bis(2,3-epithiopropylthiometh yl)-3,6-dithiaoctane, 1,8-bis(2,3-epithiopropylthio)-2,4,5-tris(2,3-epithiopropylthiom ethyl)-3,6-dithiaoctane, 1,1,1-tris[[2-(2,3-epithiopropylthio) ethyl]thiomethyl]-2-(2,3-epithiopropylthio) ethane, 1,1,2,2-tetrakis[[2-(2,3-epithiopropylthio) ethyl]thiomethyl] ethane, 1,11-bis(2,3 epithiopropylthio)-4,8-bis(2,3-epithiopropylthiomethyl)-3,6,9-tr ithiaundecane, 1,11-bis(2,3-epithiopropylthio)-4,7-bis(2,3-epithiopropylthiomet hyl)-3,6,9-trithiaundecane, and 1,11-bis(2,3-epithiopropylthio)-5,7-bis(2,3-epithiopropylthiomet hyl)-3,6,9-trithiaundecane;
cyclic aliphatic 2,3-epithiopropylthio compounds such as 1,3-bis(2,3-epithiopropylthio) cyclohexane, 1,4-bis(2,3-epithiopropylthio) cyclohexane, 1,3-bis(2,3-epithiopropylthiomethyl) cyclohexane, 1,4-bis(2,3-epithiopropylthiomethyl) cyclohexane, 2,5-bis(2,3-epithiopropylthiomethyl)-1,4-dithiane, 2,5-bis[[2-(2,3-epithiopropylthio) ethyl] thiomethyl]-1,4-dithiane, and 2,5-bis(2,3-epithiopropylthiomethyl)-2,5-dimethyl-1,4-dithiane;
aromatic 2,3-epithiopropylthio compounds such as 1,2-bis(2,3-epithiopropylthio) benzene, 1,3-bis(2,3-epithiopropylthio) benzene, 1,4-bis(2,3-epithiopropylthio) benzene, 1,2-bis(2,3-epithiopropylthiomethyl) benzene, 1,3-bis(2,3-epithiopropylthiomethyl) benzene, 1,4-bis(2,3-epithiopropyl thiomethyl) benzene, bis[4-(2,3-epithiopropylthio) phenyl] methane, 2,2-bis[4-(2,3-epithiopropylthio) phenyl] propane, bis[4-(2,3-epithiopropylthio) phenyl] sulfide, bis[4-(2,3-epithiopropylthio) phenyl] sulfone, and 4,4'-bis(2,3-epithiopropylthio) biphenyl; compounds having one epithio group such as ethylene sulfide, propylene sulfide, mercaptopropylene sulfide, mercaptobutene sulfide, and epithiochlorohydrin;
chain aliphatic 2,3-epithiopropyloxy compounds such as bis(2,3-epithiopropyl) ether, bis(2,3-epithiopropyloxy) methane, 1,2-bis(2,3-epithiopropyloxy) ethane, 1,2-bis(2,3-epithiopropyloxy) propane, 1,3-bis(2,3-epithiopropyloxy) propane, 1,3-bis(2,3-epithiopropyloxy)-2-methyl propane, 1,4-bis(2,3-epithiopropyloxy) butane, 1,4-bis(2,3-epithiopropyloxy)-2-methylbutane, 1,3-bis(2,3-epithiopropyloxy) butane, 1,5-bis(2,3-epithiopropyloxy) pentane, 1,5-bis(2,3-epithiopropyloxy)-2-methylpentane, 1,5-bis(2,3-epithiopropyloxy)-3-thiahexane, 1,6-bis(2,3-epithiopropyloxy) hexane, 1,6-bis(2,3-epithiopropyloxy)-2-methylhexane, 3,8-bis(2,3-epithiopropyloxy)-3,6-dithiaoctane, 1,2,3-tris(2,3-epithiopropyloxy) propane, 2,2-bis(2,3-epithiopropyloxy)-1,3-bis(2,3-epithiopropyloxymethyl ) propane, 2,2-bis(2,3-epithiopropyloxymethyl)-1-(2,3-epithiopropyloxy) butane, 1,5-bis(2,3-epithiopropyloxy)-2-(2,3-epithiopropyloxymethyl)-3-t hiapentane, 1,5-bis(2,3-epithiopropyloxy)-2,4-bis(2,3-epithiopropyloxymethyl )-3-thiapentane, 1-(2,3-epithiopropyloxy)-2,2-bis(2,3-epithiopropyloxymethyl)-4-t hiapentane, 1,5,6-tris(2,3-epithiopropyloxy)-4-(2,3-epithiopropyloxymethyl)-3-thiahexane, 1,8-bis(2,3-epithiopropyloxy)-4-(2,3-epithiopropyloxymethyl)-3,6 -dithiaoctane, 1,8-bis(2,3-epithiopropyloxy)-4,5-bis(2,3-epithiopropyloxymethyl )-3,6-dithiaoctane, 1,8-bis(2,3-epithiopropyloxy)-4,4-bis(2,3-epithiopropyloxymethyl )-3,6-dithiaoctane, 1,8-bis(2,3-epithiopropyloxy)-2,5-bis(2,3-epithiopropyloxymethyl )-3,6-dithiaoctane, 1,8-bis(2,3-epithiopropyloxy)-2,4,5-tris(2,3-epithiopropyloxymet hyl)-3,6-dithiaoctane, 1,1,1-tris[[2-(2,3-epithiopropyloxy) ethyl] thiomethyl]-2-(2,3-epithiopropyloxy) ethane, 1,1,2,2-tetrakis[[2-(2,3-epithiopropyloxy) ethyl]thiomethyl] ethane, 1,11-bis(2,3-epithiopropyloxy)-4,8-bis(2,3-epithiopropyloxymethy l)-3,6,9-trithiaundecane, 1,11-bis(2,3-epithiopropyloxy)-4,7-bis(2,3-epithiopropyloxymethy l)-3,6,9-trithiaundecane, and 1,11-bis(2,3-epithiopropyloxy)-5,7-bis(2,3-epithiopropyloxymethy l)-3,6,9-trithiaundecane;

cyclic aliphatic 2,3-epithiopropyloxy compounds such as 1,3-bis(2,3-epithiopropyloxy) cyclohexane, 1,4-bis(2,3-epithiopropyloxy) cyclohexane, 1,3-bis(2,3-epithiopropyloxymethyl) cyclohexane, 1,4-bis(2,3-epithiopropyloxymethyl) cyclohexane, 2,5-bis(2,3-epithiopropyloxymethyl)-1,4-dithiane, 2,5-bis[[2-(2,3-epithiopropyloxy) ethyl] thiomethyl]-1,4-dithiane, and 2,5-bis(2,3-epithiopropyloxymethyl)-2,5-dimethyl-1,4-dithiane;

aromatic 2,3-epithiopropyloxy compounds such as 1,2-bis(2,3-epithiopropyloxy) benzene, 1,3-bis(2,3-epithiopropyloxy) benzene, 1,4-bis(2,3-epithiopropyloxy) benzene, 1,2-bis(2,3-epithiopropyloxymethyl) benzene, 1,3-bis(2,3-epithiopropyloxymethyl) benzene, 1,4-bis(2,3-epithiopropyloxymethyl) benzene, bis[4-(2,3-epithiopropyloxy) phenyl] methane, 2,2-bis[4-(2,3-epithiopropyloxy) phenyl] propane, bis[4-(2,3-epithiopropyloxy) phenyl] sulfide, bis[4-(2,3-epithiopropyloxy) phenyl] sulfone, and 4,4'-bis(2,3-epithiopropyloxy) biphenyl. These episulfides may be used alone, or two or more may be used in combination.

The episulfide (b) preferably includes a compound having two episulfide groups in the molecule and more preferably includes a compound represented by General Formula (1).

The compound represented by General Formula (1) is specifically bis(2,3-epithiopropyl)sulfide or bis(2,3-epithiopropyl)disulfide. This compound can be used singly, or two or more compounds can also be used in a mixture form.

The episulfide represented by General Formula (1) can be synthesized using a well-known method. Specifically, the episulfide can be synthesized using the following synthesis route I (reaction scheme (2)) or synthesis route II (reaction scheme (3)).

(Synthesis route I)

The synthesis route I is made up of the following steps. Meanwhile, in the respective steps, well-known raw materials, well-known reaction conditions, and the like can be employed as raw materials used, reaction conditions, and the like, and there are no particular limitations.

(i) A step in which epichlorohydrin is reacted with a sulfating agent, thereby obtaining bischlorohydrin and then a bis(chlorohydrin)sulfide compound (a) in the following reaction scheme (2))

(ii) A step in which the bis(chlorohydrin)sulfide compound is epoxidized under base conditions, thereby obtaining an epoxy compound (b) in the following reaction scheme (2))

(iii) A step in which the epoxy compound is reacted with a sulfating agent, thereby obtaining bis(2,3-epithiopropyl)sulfide (c) in the following reaction scheme (2))

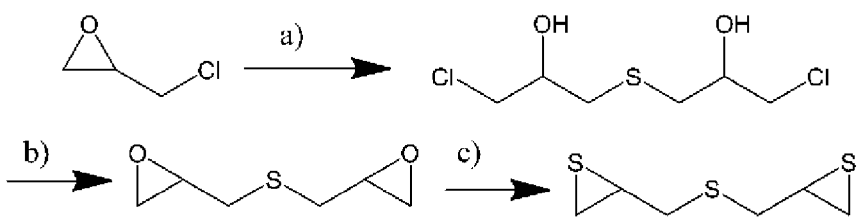


(2)

(Synthesis route II)

The synthesis route II is made up of the following steps. Meanwhile, in the respective steps, well-known raw

materials, well-known reaction conditions, and the like can be employed as raw materials used, reaction conditions, and the like, and there are no particular limitations.

(i) A step in which epichlorohydrin is reacted with a sulfating agent and then is oxidized, thereby obtaining a bis(chlorohydrin)disulfide compound (a) in the following reaction scheme (3))
(ii) A step in which the bis(chlorohydrin)disulfide compound is epoxidized under base conditions, thereby obtaining an epoxy compound (b) in the following reaction scheme (3))
(iii) A step in which the epoxy compound is reacted with a sulfating agent, thereby obtaining bis(2,3-epithiopropyl)disulfide (c) in the following reaction scheme (3))

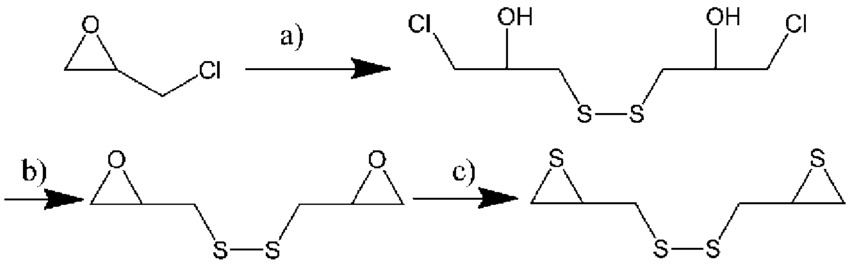

(3)

In the above-described reactions, when the raw material of epichlorohydrin is changed to a raw material obtained from glycerin which is a plant-derived material, it is possible to obtain the episulfide (b) having high degree of biomass.
Examples of a manufacturing method for obtaining epichlorohydrin from glycerin include a manufacturing method in which glycerin is chlorinated so as to obtain dichloropropanol, and the dichloropropanol is epoxidized, thereby obtaining epichlorohydrin.
Examples of epichlorohydrin manufactured from plant-derived glycerin include EPICHLOROHYDRIN(ECH) (manufactured by Solvay) and the like.

[Polythiol (c)]

In the present embodiment, examples of the polythiol having a di- or higher-functional thiol group (c) (hereinafter, simply referred to as the polythiol (c)) include aliphatic polythiol compounds such as methanedithiol, 1,2-ethanedithiol, 1,2,3-propanetrithiol, 1,2-cyclohexanedithiol, bis(2-mercaptoethyl) thioether, tetrakis(mercaptomethyl) methane, diethylene glycol bis(2-mercaptoacetate), diethylene glycol bis(3-mercaptopropionate), ethylene glycol bis(2-mercaptoacetate), ethylene glycol bis(3-mercaptopropionate), trimethylolpropane tris(2-mercaptoacetate), trimethylolpropane tris(3-mercaptopropionate), trimethylolethane tris(2-mercaptoacetate), trimethylolethane tris(3-mercaptopropionate), pentaerythritol tetrakis mercaptoacetate, pentaerythritol tetrakis(2-mercaptoacetate), pentaerythritol tetrakis mercaptopropionate, pentaerythritol tetrakis(3-mercaptopropionate), bis(mercaptomethyl)sulfide, bis(mercaptomethyl)disulfide, bis(mercaptoethyl)sulfide, bis(mercaptoethyl)disulfide, bis(mercaptopropyl)sulfide, bis(mercaptomethylthio) methane, bis(2-mercaptoethylthio) methane, bis(3-mercaptopropylthio) methane, 1,2-bis(mercaptomethylthio) ethane, 1,2-bis(2-mercaptoethylthio) ethane, 1,2-bis(3-mercaptopropylthio) ethane, 1,2,3-tris(mercaptomethylthio)propane, 1,2,3-tris(2-mercaptoethylthio) propane, 1,2,3-tris(3-mercaptopropylthio) propane, 4-mercaptomethyl-1,8-dimercapto-3,6-dithiaoctane, 3-mercaptomethyl-1,5-dimercapto-2,4-dithiapentane, 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane, tetrakis(mercaptomethylthiomethyl) methane, tetrakis(2-mercaptoethylthiomethyl) methane, tetrakis(3-mercaptopropylthiomethyl) methane, bis(2,3-dimercaptopropyl)sulfide, 2,5-dimercaptomethyl-1,4-dithiane, 2,5-dimercapto-1,4-dithiane, 2,5-dimercaptomethyl-2,5-dimethyl-1,4-dithiane, esters of thioglycolic acids and mercaptopropionic acids thereof, hydroxymethyl sulfide bis(2-mercaptoacetate), hydroxymethyl sulfide bis(3-mercaptopropionate), hydroxyethyl sulfide bis(2-mercaptoacetate), hydroxyethyl sulfide bis(3-mercaptopropionate), hydroxymethyl disulfide bis(2-mercaptoacetate), hydroxymethyl disulfide bis(3-mercaptopropionate), hydroxyethyl disulfide bis(2-mercaptoacetate), hydroxyethyl disulfide bis(3-mercaptopropionate), 2-mercaptoethyl ether bis(2-mercaptoacetate), 2-mercaptoethyl ether bis(3-mercaptopropionate), bis(2-mercaptoethyl ester) thiodiglycolate, bis(2-mercaptoethyl ester) thiodipropionate, bis(2-mercaptoethyl ester) dithiodiglycolate, bis(2-mercaptoethyl ester) dithiodipropionate, 1,1,3,3-tetrakis(mercaptomethylthio)propane, 1,1,2,2-tetrakis(mercaptomethylthio) ethane, tris(mercaptomethylthio) methane, and tris(mercaptoethylthio) methane;
aromatic polythiol compounds such as 1,2-dimercapto benzene, 1,3-dimercapto benzene, 1,4-dimercapto benzene, 1,2-bis(mercaptomethyl) benzene, 1,3-bis(mercaptomethyl) benzene, 1,4-bis(mercaptomethyl) benzene, 1,2-bis(mercaptoethyl) benzene, 1,3-bis(mercaptoethyl) benzene, 1,4-bis(mercaptoethyl) benzene, 1,3,5-trimercapto benzene, 1,3,5-tris(mercaptomethyl) benzene, 1,3,5-tris(mercaptomethyleneoxy) benzene, 1,3,5-tris(mercaptoethyleneoxy) benzene, 2,5-toluene dithiol, 3,4-toluene dithiol, 1,5-naphthalene dithiol, and 2,6-naphthalene dithiol;

heterocyclic polythiol compounds such as 2-methylamino-4,6-dithiol-sym-triazine, 3,4-thiophene dithiol, bismuthiol, 4,6-bis(mercaptomethylthio)-1,3-dithiane, and 2-(2,2-bis(mercaptomethylthio) ethyl)-1,3-dithietane, and at least one polythiol compound can be used.

Furthermore, it is also possible to use an oligomer of the polythiol compound or a halogen-substituted body such as a chlorine-substituted body or a bromine-substituted body.

As the polythiol (c), it is possible to preferably use at least one selected from 4-mercaptomethyl-1,8-dimercapto-3,6-dithiaoctane, 4,8 or 4,7 or 5,7-dimercaptomethyl-1,11-dimercapto-3,6,9-trithiaundecane, pentaerythritol tetrakis mercaptoacetate, pentaerythritol tetrakis(2-mercaptoacetate), pentaerythritol tetrakis mercaptopropionate, pentaerythritol tetrakis(3-mercaptopropionate), 2,5-bis(mercaptomethyl)-1,4-dithiane, bis(mercaptoethyl)sulfide, 1,1,3,3-tetrakis(mercaptomethylthio)propane, 4,6-bis(mercaptomethylthio)-1,3-dithiane, 2-(2,2-bis(mercaptomethylthio) ethyl)-1,3-dithietane, 1,1,2,2-tetrakis(mercaptomethyl) ethane, 3-mercaptomethyl-1,5-dimercapto-2,4-dithiapentane, tris(mercaptomethylthio) methane, and ethylene glycol bis(3-mercaptopropionate).

Furthermore, among these, a polythiol obtained from plant-derived materials is more preferably used.

Examples of the polythiol obtained from plant-derived materials include 4-mercaptomethyl-1,8-dimercapto-3,6-dithiaoctane and 4,8 or 4,7 or 5,7-dimercaptomethyl-1,11-dimercapto-3,6,9-trithiaundecane which are synthesized using epichlorohydrin manufactured by chlorinating and epoxidizing glycerin obtained from plant-derived materials as a raw material, and one or more polythiols among the above-described polythiols can be used. Meanwhile, the glycerin can be obtained by extracting glycerin from an aliphatic acid ester of glycerin included in vegetable oil and fat such as canola oil, palm oil, castor oil, or olive oil by means of hydrolysis or ester exchange.

For example, in a case of the 4-mercaptomethyl-1,8-dimercapto-3,6-dithiaoctane, it can be manufactured through a) a step of adding two molecules of 2-mercaptoethanol into epichlorohydrin manufactured from naturally originated glycerin, preferably plant-derived glycerin, through chlorination and epoxidation, and b) a step of synthesizing 4-mercaptomethyl-1,8-dimercapto-3,6-dithiaoctane through a decomposition step of a base which is continuous to a reaction with respect to a sulfurizing agent such as thiourea.

By using the polythiol obtained from plant-derived material in the polymerizable composition, it is possible to provide a molding formed of a polythiourethane resin having a high degree of biomass of greater than or equal to 25% and excellent properties of transparency, a refractive index, heat resistance, strength, and the like.

It is preferable that the polythiol (b) to be used is obtained from the plant-derived material, and a compound which is not obtained from the plant-derived material is also may be used. As it is used, the compound is may be used such that the degree of biomass of the polythiourethane resin is in a predetermined range (greater than or equal to 25%).

(Polymerization catalyst)

When the polymerizable composition of the present embodiment is heated or left to stand at normal temperature in the presence or absence of a curing catalyst so as to be polymerized, it is possible to manufacture resins.

As the kinds of the present polymerization catalyst, amines, phosphines, organic acids and salts thereof, esters, anhydrides, inorganic acids, quaternary ammonium salts, quaternary phosphonium salts, tertiary sulfonium salts, secondary iodonium salts, Lewis acids, radical polymerization catalysts, cationic polymerization catalysts, and the like are generally used.

The polymerization catalyst may be used singly, or two or more polymerization catalysts may be used in a mixture form, and, when two or more polymerization catalysts having different reactive properties are jointly used out of the above-described polymerization catalysts, there are cases in which the handling properties of monomers and the optical properties, hue, transparency, and optical strain (striae) of resins being obtained improve, and thus there are preferable cases.

Specific examples of preferred curing catalysts include quaternary ammonium salts such as tetramethylammonium bromide, tetraethylammonium bromide, tetrapropylammonium bromide, tetrabutylammonium bromide, tetrahexylammonium bromide, and tetraethyl ammonium hydroxide, for example, imidazoles such as imidazole, 1,2-dimethyl imidazole, benzyl methyl imidazole, and 2-ethyl-4-imidazole, for example, pyrazoles such as pyrazole, 3,5-dimethylpyrazole, for example, hindered amines such as 1,2,2,6,6-pentamethyl-4-piperidinol, 1,2,2,6,6-pentamethyl-4-hydroxyethyl-4-piperidinol, methyl-1,2,2,6,6-pentamethyl-4-piperidyl sebacate, mixtures of methyl-1,2,2,6,6-pentamethyl-4-piperidyl sebacate and bis(1,2,2,6,6-pentamethyl-4-piperidyl) sebacate, bis(1,2,2,6,6-pentamethyl-4-piperidyl) sebacate, bis(2,2,6,6-tetramethyl-1-(octyloxy)-4-piperidyl) sebacate, bis(1,2,2,6,6-pentamethyl-4-piperidyl)[[3,5-bis(1,1-dimethylethy l)-4-hydroxyphenyl]methyl] butylmalonate, and tetrakis(1,2,2,6,6-pentamethyl-4-piperidyl) butane-1,2,3,4-tetracarboxylate. Additionally, examples thereof include phosphines such as trimethylphosphine, triethylphosphine, tri-n-propylphosphine, triisopropylphosphine, tri-n-butylphosphine, triphenylphosphine, tribenzylphosphine, 1,2-bis(diphenylphosphino) ethane, and 1,2-bis(dimethylphosphino) ethane, Lewis acids such as dimethyl tin dichloride, dibutyl tin

dichloride, dibutyl tin dilaurate, tetrachloro tin, dibutyl tin oxide, zinc chloride, zinc acetylacetone, aluminum chloride, aluminum fluoride, triphenyl aluminum, tetrachlorotitanium, and calcium acetate, and cationic polymerization catalysts such as diphenyliodonium hexafluorophosphate, diphenyliodonium hexafluoroarsenate, diphenyliodonium hexafluoroantimonate, triphenylsulfonium tetrafluoroborate, triphenylsulfonium hexafluorophosphate, and triphenylsulfonium hexafluoroarsenate, but the curing catalyst is not limited to these exemplary compounds.

The curing catalysts may be used alone, or two or more may be used in combination.

The amount of the curing catalyst added is within a range of 0.001 wt% to 10 wt% and preferably within a range of 0.01 wt% to 1 wt% of the total weight of the polymerizable compound.

(Sulfur)

To the polymerizable composition of the present embodiment, it is possible to add sulfur in order to improve the refractive index of moldings to be obtained. For sulfur that is used for optical resins, the purity thereof is preferably higher than or equal to 98%, more preferably higher than or equal to 99%, and still more preferably higher than or equal to 99.5%. In order to increase the purity, there are cases in which a method for removing a volatile component is also preferred.

Regarding the properties, sulfur may have any forms as long as the sulfur can be dissolved in the polymerizable composition, but a powder form is preferred, and a fine powder form is more preferred.

In a case in which the total weight of the episulfide compound and sulfur is set to 100 parts by weight, the amount of sulfur added is in a range of 10 parts by weight to 50 parts by weight, preferably in a range of 10 parts by weight to 40 parts by weight, and more preferably in a range of 10 parts by weight to 30 parts by weight. During addition, a well-known vulcanization catalyst such as an imidazole or an amine can be jointly used.

(Resin modifier)

Examples of a resin modifier include epoxy compounds, olefins including (meth)acrylates, amino compounds, thiol compounds, polyphenols, amino acid and mercaptoamines, organic acids, anhydrids, and mercapto organic acids.

(Other additives)

In addition to the resin modifier, a variety of well-known additives such as an internal mold release agent, a light stabilizer, a bluing agent, an ultraviolet absorber, an antioxidant, a dye, and a filler may be used depending on the purpose as long as no problems are caused. As the internal mold release agent, an acidic phosphoric acid ester represented by Formula (4) can be used.

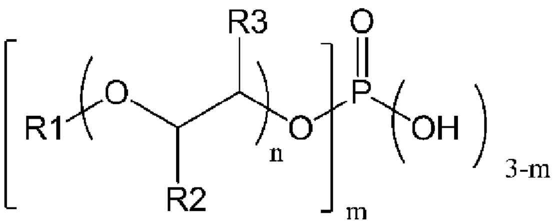 (4)

In formula (4), m represents an integer of 1 or 2, n represents an integer of 0 to 18, R1 represents an alkyl group having 1 to 20 carbon atoms, and each of R2 and R3 independently represents a hydrogen atom, a methyl group, or an ethyl group. The number of carbon atoms in $[]_m$ is preferably 4 to 20.

Examples of R1 in General Formula (4) include organic residues derived from a linear aliphatic compound such as methane, ethane, propane, butane, pentane, hexane, heptane, octane, nonane, decane, undecane, dodecane, tetradecane, and hexadecane;

organic residues derived from a branched aliphatic compound such as 2-methylpropane, 2-methylbutane, 2-methylpentane,3-methylpentane, 3-ethylpentane, 2-methylhexane, 3-methylhexane, 3-ethylhexane, 2-methylheptane, 3-methylheptane 4-methylheptane, 3-ethylheptane, 4-ethylheptane, 4-propylheptane, 2-methyloctane, 3-methyloctane, 4-methyloctane, 3-ethyloctane, 4-ethyloctane, and 4-propyloctane;

organic residues derived from alicyclic compounds such as cyclopentane, cyclohexane, 1,2-dimethylcyclohexane,1,3-dimethylcyclohexane, and 1, 4-dimethylcyclohexane; and the like, but R1 is not limited to these exemplary compounds.

Examples of commercially available products of the acidic phosphoric acid ester include ZelecUN manufactured by Stepan Company, JP series manufactured by Johoku Chemical Co., Ltd., PHOSPHANOL series manufactured by

Toho Chemical Industry Co., Ltd., AP and DP series manufactured by Daihachi Chemical Industry Co., Ltd., and the like.

As the light stabilizer, hindered amine-based compounds can be used.

Examples of the hindered amine-based compounds include Lowilite 76 and Lowilite 92 manufactured by Chemtura Corporation, Tinuvin 123, Tinuvin 144, Tinuvin 292, Tinuvin 765, and Tinuvin 770DF manufactured by BASF, ADEKASTAB LA-52 and LA-72 manufactured by ADEKA Corporation, JF-90 and JF-95 manufactured by Johoku Chemical Co., Ltd., and the like.

Examples of the bluing agent include bluing agents which have an absorption band in a wavelength range from orange color to yellow color in the visible light range and have a function of adjusting the hues of optical materials formed of a resin. More specific examples of the bluing agent include substances exhibiting blue color through violet color.

Examples of the ultraviolet absorber include benzophenone-based ultraviolet absorbers such as 2,2'-dihydroxy-4-methoxybenzophenone, 2-hydroxy-4-acryloyloxy benzophenone, 2-hydroxy-4-acryloyloxy-5-tert-butyl benzophenone, and 2-hydroxy-4-acryloyloxy-2',4'-dichlorobenzophenone,

triazine-based ultraviolet absorbers such as 2-[4-[(2-hydroxy-3-dodecyloxypropyl)oxy]-2-hydroxyphenyl]4,6-bis (2,4-dimethylphenyl)-1,3,5-triazine, 2-[4-(2-hydroxy-3-tridecyloxypropyl)oxy]-2-hydroxyphenyl]-4,6-bi s(2,4-dimethylphenyl)-1,3,5-triazine, 2-[4-[(2-hydroxy-3-(2'-ethyl)hexyl)oxy]-2-hydroxyphenyl]-4,6-bis (2,4-dimethylphenyl)-1,3,5-triazine, 2,4-bis(2-hydroxy-4-butyloxyphenyl)-6-(2,4-bis-butyloxyphenyl)-1 ,3,5-triazine, and 2-(2-hydroxy-4-[1-octyloxycarbonylethoxy] phenyl)-4,6-bis(4-phenylphenyl)-1,3,5-triazine; and

benzotriazole-based ultraviolet absorbers such as 2-(2H-benzotriazol-2-yl)-4-methylphenol, 2-(2H-benzotriazol-2-yl)-4-tert-octylphenol, 2-(2H-benzotriazol-2-yl)-4,6-bis(1-methyl-1-phenylethyl) phenol, 2-(2H-benzotriazol-2-yl)-4,6-di-tert-pentylphenol, 2-(5-chloro-2H-benzotriazol-2-yl)-4-methyl-6-tert-butylphenol, 2-(5-chloro-2H-benzotriazol-2-yl)-2,4-tert-butylphenol, and 2,2'-methylenebis[6-(2H-benzotriazol-2-yl)-4-(1,1,3,3-tetramethy lbutyl) phenol] ; and the like, and preferred examples thereof include benzotriazole-based ultraviolet absorbers such as 2-(2H-benzotriazol-2-yl)-4-tert-octylphenol and 2-(5-chloro-2H-benzotriazol-2-yl)-4-methyl-6-tert-butylphenol. These ultraviolet absorbers may be used alone, or two or more may be used in combination.

The amount of these additives is preferably within a range of 0.05 parts by weight to 2.0 parts by weight and more preferably within a range of 0.05 parts by weight to 1.5 parts by weight of a total of 100 parts by mass of the constituent components (a) to (c).

The polymerizable composition for an optical material of the present embodiment can be obtained by mixing the above-described components. Regarding the method for mixing, a well-known method in the related art can be used for mixing the components.

In the polymerizable composition for an optical material of the present embodiment, the molar ratio of all mercapto groups in the polythiol (c) to all isocyanate groups in the polyisocyanate (a) is within the range between 0.7 and 1.5, preferably the range between 0.80 and 1.3, and more preferably the range between 0.85 and 1.2.

In the above-described range, it is possible to obtain a polymerizable composition for an optical material that is preferably used as an optical material, particularly, a spectacle lens.

Effects in a preferred aspect of the present invention will be described below.

As described above, in recent years, there has been a demand for the development of biomass products produced in consideration of influences on the global environment. In order to improve the degree of biomass, there is a need for utilizing aliphatic polyisocyanate that can be manufactured from plant-derived materials. However, aliphatic polyisocyanate obtained from plant-derived materials has a chain portion that is long enough to have 5 to 18 carbon atoms, and for resin moldings obtained using the above-described aliphatic polyisocyanate, there has been room for improvement in heat resistance.

In the present invention, when the polyisocyanate (a) including aliphatic polyisocyanate (a1) having 5 to 18 carbon atoms, which is obtained from plant-derived materials, and the modified aliphatic polyisocyanate (a2), the episulfide (b), and the polythiol (c) are included, it is possible to improve heat resistance while maintaining a high level of the degree of biomass, and furthermore, it is possible to obtain a polymerizable composition for an optical material which can be used to provide moldings being excellent in terms of other properties such as transparency, refractive index, and dyeing properties.

[Molding]

When the polymerizable composition for an optical material of the present embodiment is polymerization-cured, it is possible to obtain moldings having excellent properties such as transparency, refractive index, and heat resistance.

Furthermore, when a plant-derived material is used, it is possible to obtain moldings including a resin for an optical material which are in harmony with the global environment. The degree of biomass of the resin for an optical material can be set to higher than or equal to 25%, preferably set to higher than or equal to 30%, and more preferably set to higher than or equal to 50%.

From the viewpoint of utilizing non-fossil resources, it is preferable to use a material for which a plant-derived

material is used and which has high degree of biomass. However, as the degree of biomass of a resin is improved, there have been cases in which properties and the like degrade.

As a result of intensive studies, the present inventors found that, when a compound obtained from at least one plant-derived material selected from the polyisocyanate (a), the episulfide (b), and the polythiol (c) which are obtained from plant-derived materials is used, a resin for an optical material having a degree of biomass of higher than or equal to 25% and a molding including the resin can be obtained, and a resin for an optical material being excellent in terms of transparency and heat resistance and also having an excellent balance among properties such as refractive index, Abbe number, mold release properties, and the like and a molding including the resin can be obtained and the present inventors completed the present invention.

The glass transition temperature (Tg) of the molding is higher than or equal to 60°C, preferably higher than or equal to 70°C, and more preferably higher than or equal to 80°C. The refractive index is higher than or equal to 1.60, preferably higher than or equal to 1.63, and more preferably higher than or equal to 1.67.

[Use]

Optical materials formed of the molding of the present embodiment can be provided with a variety of shapes by changing molds during casting polymerization. Specifically, the optical materials can be used in a variety of uses such as plastic lenses, camera lenses, light-emitting diodes (LEDs), prisms, optical fibers, information-recording substrates, filters, light-emitting diodes, optical lenses for vehicles, and optical lenses for robots. Particularly, the optical materials are preferred as optical materials and optical elements such as plastic lenses, camera lenses, and light-emitting diodes.

Examples of plastic lenses include plastic spectacle lenses formed of a resin molding for an optical material and plastic polarizing lenses in which a layer formed of the resin molding is laminated on at least one surface of a polarizing film.

[Method for manufacturing plastic spectacle lens]

A method for manufacturing a plastic spectacle lens of the present embodiment includes the following steps.

Step (1) : Injecting the polymerizable composition for an optical material of the embodiment into a lens casting mold.

Step (2) : Polymerizing and curing the polymerizable composition for an optical material in the lens casting mold.

Hereinafter, the respective steps will be sequentially described.

Step (1)

In this step, the polymerizable composition of the embodiment is injected into a molding mold (the lens casting mold) held by a gasket, tape, or the like. At this time, it is preferable that, as necessary, a defoaming treatment under reduced pressure, a filtration treatment such as pressurization and depressurization, or the like, is performed according to the properties required to a molding to be obtained.

Step (2)

In this step, polymerization of the polymerizable composition casted in the casting mold at a predetermined temperature is initiated, and the composition is polymerized. The polymerization conditions are considerably different based on the kinds of polyisocyanate or polythiols to be used, the shape of the mold, and the like, and thus, the conditions are not limited, and the polymerization is performed at a temperature of 0°C to 140°C for 1 hour to 48 hours.

The obtained plastic spectacle lens may be used after being provided with a coating layer (s) on one or both surface (s) as necessary. Examples of the coating layer include a primer layer, a hard coat layer, an antireflection layer, an antifogging coating layer, an antifouling layer, and a water-repellent layer. Each of these coating layers may be used singly, or a plurality of the coating layers may be used in a multilayer form. In a case in which the coating layers are provided on both surfaces, the same coating layers or different coating layers may be provided on the respective layers.

With these coating layers, an ultraviolet absorber for the purpose of protecting the lens or eyes from ultraviolet rays, an infrared absorber for the purpose of protecting eyes from infrared rays, a light stabilizer or an antioxidant for the purpose of improving the weather resistance of the lens, dyes or pigments and, furthermore, photochromic dyes or photochromic pigments for the purpose of enhancing the fashion properties of the lens, an antistatic agent, and, additionally, well-known additives for enhancing the performance of the lens may be jointly used. For layers coated by means of coating, a variety of levelling agents intended to improve coating properties may be used.

The primer layer is generally formed between a hard coat layer described below and an optical lens. The primer

layer is a coating layer intended to improve adhesiveness to the hard coat layer and the lens which are formed on the primer layer and, in some cases, is also capable of improving impact resistance. Any material can be used for the primer layer as long as the material is highly adhesive to the obtained optical lens; however, generally, primer compositions including a urethane-based resin, an epoxy-based resin, a polyester-based resin, a melamine-based resin, and polyvinyl acetal as main components and the like are used. In the primer composition, an appropriate solvent having no influences on the lens may be used in order to adjust the viscosity of the composition. It is needless to say that no solvents may be used.

The primer composition can also be formed using any one of a coating method and a dry method. In a case in which a coating method is used, the primer layer is formed by applying the primer composition onto a lens using a well-known coating method such as spin coating or dip coating and then solidifying the primer composition. In a case in which a dry method is used, the primer layer is formed using a well-known dry method such as a CVD method or a vacuum deposition method. During the formation of the primer layer, the surface of the lens may be subj ected to a pretreatment such as an alkali treatment, a plasma treatment, or an ultraviolet treatment as necessary in order to improve adhesiveness.

The hard coat layer is a coating layer intended to impart functions such as abrasion resistance, wear resistance, moisture resistance, hot water resistance, heat resistance, and weather resistance to the lens surface.

For the hard coat layer, generally, a hard coat composition including one or more kinds of fine particles constituted of a complex oxide of a curable organic silicon compound and one or more kinds of oxide fine particles of an element selected from an element group of Si, Al, Sn, Sb, Ta, Ce, La, Fe, Zn, W, Zr, In, and Ti and/or two or more kinds of elements selected from the above-described element group is used.

The hard coat composition preferably includes, in addition to the above-described components, at least any of amines, amino acids, metallic acetylacetonate complexes, organic acid metallic salts, perchloric acids, salts of perchloric acids, acids, metal chlorides, and polyfunctional epoxy compounds. In the hard coat composition, an appropriate solvent having no influences on the lens may be used. It is needless to say that the hard coat composition may include no solvents.

The hard coat layer is generally formed by applying the hard coat composition using a well-known coating method such as spin coating or dip coating and then curing the hard coat composition. Examples of a curing method include curing methods in which the hard coat composition is cured by means of thermal curing or irradiation with energy rays such as ultraviolet rays or visible light rays. In order to suppress the generation of interference stripes, the refractive index of the hard coat layer is preferably in a range of ± 0.1 from the refractive index of the lens.

Generally, the antireflection layer is formed on the hard coat layer as necessary. Antireflection layers are classified into inorganic antireflection layers and organic antireflection layers, and, in a case in which the antireflection layer is an inorganic antireflection layer, the antireflection layer is formed using an inorganic oxide such as $SiO_2$ or $TiO_2$ and a dry method such as a vacuum deposition method, a sputtering method, an ion plating method, an ion beam assist method, or a CVD method. In a case in which the antireflection layer is an organic antireflection layer, the antireflection layer is formed using a composition including an organic silicon compound and silica-based fine particles having internal voids and a wet method.

The antireflection layer may have a single layer structure or a multilayer structure, and, in a case in which a single layer-structured antireflection layer is used, the refractive index of the antireflection layer is preferably lower than the refractive index of the hard coat layer by at least greater than or equal to 0.1. In order to effectively develop an antireflection function, a multilayered antireflection film is preferably formed, and, in this case, low-refractive index films and high-refractive index films are alternately laminated. In this case as well, the difference in the refractive index between the low-refractive index film and the high-refractive index film is preferably greater than or equal to 0.1. Examples of the high-refractive index films include films of ZnO, $TiO_2$, $CeO_2$, $Sb_2O_5$, $SnO_2$, $ZrO_2$, and $Ta_2O_5$, and examples of the low-refractive index films include $SiO_2$ films and the like.

On the antireflection layer, an antifogging coating layer, an antifouling layer, and a water-repellent layer may be formed as necessary. Regarding methods for forming the antifogging coating layer, the antifouling layer, and the water-repellent layer, treatment methods, treatment materials, and the like are not particularly limited as long as there are no adverse effects on the antireflection function, and well-known antifogging coating treatment methods, antifouling treatment methods, and water-repellent treatment methods, and materials can be used. Examples of antifogging coating and antifouling treatment methods include a method in which the surface is covered with a surfactant, a method in which a hydrophilic film is attached to the surface so as to impart water-absorbing properties, a method in which the surface is covered with fine protrusion and recesses so as to enhance water-absorbing properties, a method in which water-absorbing properties are imparted using a photocatalytic activity, a method in which a superhydrophobic treatment is carried out so as to prevent the attachment of water droplets, and the like. In addition, examples of the water-repellent treatment include a method in which a water-repellent treatment layer is formed by depositing or sputtering a fluorine-containing silane compound or the like, a method in which a fluorine-containing silane compound is dissolved in a solvent and then is applied, thereby forming a water-repellent treatment layer, and the like.

The plastic spectacle lens of the present embodiment may be dyed using appropriate colorants in order to impart fashion properties or photochromic properties. The lens can be dyed using a well-known dyeing method and is

dyed using, for example, the following method.

Generally, a lens raw material finished to a predetermined optical surface is immersed in a dyeing fluid in which a colorant being used is dissolved or uniformly dispersed (dyeing step), and then the colorant is fixed by heating the lens as necessary (post-dyeing annealing step). Colorants that are used in the dyeing step are not particularly limited as long as the colorants are well-known colorants; however, generally, oil-soluble dyes or dispersive dyes are used. The solvent that is used in the dyeing step is not particularly limited as long as the solvent is capable of dissolving or uniformly dispersing colorants being used. In this dyeing step, a surfactant for dispersing the colorants in the dyeing fluid or a carrier for accelerating dyeing may be added as necessary. In the dyeing step, colorants and a surfactant, which is added as necessary, are dispersed in water or a mixture of water and an organic solvent so as to prepare a dyeing bath, an optical lens is immersed in this dyeing bath, and dyeing is carried out at a predetermined temperature for a predetermined time. The dyeing temperature and the dyeing time vary depending on desire coloration densities; however, generally, the dyeing temperature and the dyeing time are preferably lower than or equal to 120°C and approximately several minutes to several tens of hours, and the dyeing density of the dyeing bath is within a range of 0.01% by weight to 10% by weight. In addition, in a case in which dyeing is difficult, the lens may be dyed under pressurization. The post-dyeing annealing step which is carried out as necessary is a step in which a heating treatment is carried out on the dyed lens material. In the heating treatment, water remaining on the surface of the lens material dyed in the dyeing step is removed using a solvent or the like or the solvent is dried with wind, and then the lens material is retained in, for example, a furnace such as an infrared heating furnace in the atmosphere or a resistance heating furnace for a predetermined time. The post-dyeing annealing step prevents the discoloration of the dyed lens material (discoloration prevention step) and removes moisture that has intruded into the lens material during dyeing.

[Method for manufacturing plastic polarizing lens]

A method for manufacturing a plastic polarizing lens of the present embodiment includes the following steps.

Step (a): Fixing a polarizing film into a lens casting mold in a state where at least one surface of the polarizing film is separated from a mold.

Step (b) : Injecting the polymerizable composition for an optical material of the embodiment into a gap between the polarizing film and the mold.

Step (c) : Laminating a layer formed of a polythiourethane resin on at least one surface of the polarizing film by polymerizing and curing the polymerizable composition for an optical material.

Hereinafter, the respective steps will be sequentially described.

Step (a)

The polarizing film is disposed in a space of the lens casting mold such that at least one surface of the film is parallel to the facing inner surface of the mold. A gap portion is formed between the polarizing film and the mold. The polarizing film may be formed in advance.

Various films such as a polyvinyl alcohol polarizing film and a thermoplastic polyester polarizing film can be used in the polarizing film. Examples of the thermoplastic polyester may include polyethylene terephthalate, polyethylene naphthalate, polybutylene terephthalate, and the like.

Specifically, examples of the polarizing film include a dichromic dye-containing thermoplastic polyester polarizing film, an iodine-containing polyvinyl alcohol polarizing film, a dichromic dye-containing polyvinyl alcohol polarizing film, and the like.

Step (b)

Next, in the space of the lens casting mold, the polymerizable composition for an optical material of the embodiment is injected into the gap portion between the mold and the polarizing film by a predetermined injection unit.

Step (c)

Next, the lens casting mold into which the polymerizable composition for an optical material is injected and into which the polarizing film is fixed is heated in a device in which heating is may be performed, such as an oven or water according to a predetermined temperature program for several hours to several tens of hours, and thus, the molding is performed by polymerizing and curing the polymerizable composition for an optical material.

The polymerizing and curing temperature is not limited since conditions are different according to the composition of the polymerizable composition, the kinds of catalyst, the shape of the mold, and the like, but the polymerizing and curing is performed at a temperature of 0°C to 140°C for 1 hour to 48 hours.

When the polymerizable composition for an optical material is removed from the mold for lens casting after the end of the curing and molding, it is possible to obtain the plastic polarizing lens of the present embodiment in which a layer formed of a polythiourethane resin is laminated on at least one surface of the polarizing film.
For the plastic polarizing lens of the present embodiment, for the purpose of alleviating strain caused by the polymerization, it is desirable to carry out an annealing treatment by heating the released lens.
The plastic polarizing lens of the present embodiment may be used after being provided with a coating layer(s) on one or both surface(s) as necessary. Examples of the coating layer, similar to the coating layer for the plastic spectacle lens, include a primer layer, a hard coat layer, an antireflection layer, an antifogging coating layer, an antifouling layer, and a water-repellent layer.
The embodiment of the present invention has been described above, but the embodiment is an example of the present invention, and a variety of other constitutions can be employed as long as the effects of the present invention are not impaired.

[Examples]

Hereafter, the present invention will be described in more detail using examples, but the present invention is not limited thereto. In the following description, unless otherwise particularly stated, "parts" and % are based on a weight.
(Method for measuring the concentration (%) of unreacted 1,5-pentamethylene diisocyanate in polyisocyanate (1) which is a composition including a modified 1,5-pentamethylene diisocyanate)
The concentration of unreacted 1, 5-pentamethylene diisocyanate in polyisocyanate (1) including a modified product was obtained from standard curves produced using 1,5-pentamethylene diisocyanate obtained in Preparation Example 4 described below as a standard product and the following device.
Device: Prominence (manufactured by Shimadzu Corporation)
Column: SHISEIDO SILICA SG-120
Column temperature: 40°C
Eluent: n-Hexane/methanol/1,2-dichloroethane=90/5/5 (volume ratio)
Flow rate: 0.2 ml/min
Detector: UV 225 nm
R. Time: 16.9 min
Preparation of measurement solution: A sample (0.1 g) and dibenzyl amine amounting to approximately 20 times the mole of the sample were added to a 50 ml measuring flask, and the mass was increased using 1, 2-dichloroethane, thereby preparing a measurement solution.
Measurement: The measurement solution (1 μL) was injected, and the concentration was measured.

(Method for measuring the concentration (%) of isocyanate groups in polyisocyanate (1))

The concentration of an isocyanate group of the polyisocyanate was obtained by being measured by an n-dibutyl amine method using a potential difference titration device on the basis of JIS K-1556.

(Method for measuring the concentration (%) of mononuclear isocyanurate in polyisocyanate (1))

The area ratio of a peak corresponding to a molecular weight three times the molecular weight of 1, 5-pentamethylene diisocyanate to the entire peak area was obtained as the concentration (%) of mononuclear isocyanurates in polyisocyanate (1) from chromatograms obtained by means of gel permeation chromatography using the following device.
Device: HLC-8020 (manufactured by Tosoh Corporation)
Column: Direct connection of G1000HXL, G2000HXL, and G3000HXL (manufactured by Tosoh Corporation)
Column temperature: 40°C
Eluent: Tetrahydrofuran
Flow rate: 0.8 ml/min
Detector: Differential Refractometer
R. Time: Mononuclear Isocyanurate, 27.2 min
Standard substance: Polyethylene oxide (manufactured by Tosoh Corporation, TSK standard polyethylene oxide)
Measurement: The specimen (30 mg) was dissolved in tetrahydrofuran (10 ml), the obtained solution (100 μL) was injected, and the concentration was measured.

(Method for calculating the average number of functional groups in polyisocyanate (1))

The average number of functional groups in polyisocyanate (1) was calculated by the following expression using a number average molecular weight obtained by the same measurement as that of the concentration of the mononuclear isocyanurate in the polyisocyanate (1), and the concentration of isocyanate groups in polyisocyanate (1).

(The average number of functional groups in polyisocyanate (1))=(the number-average molecular weight of polyisocyanate (1))x(the concentration (%) of isocyanate groups in polyisocyanate (1))/4202

(Degree of biomass (%))

The degree of biomass is obtained by a calculation method on a carbon basis, that is, is defined as follows.

Degree of Biomass (%) = {(Number of Plant-derived Carbon Atoms) / (Number of Plant-derived Carbon Atoms + Number of Petroleum-Derived Carbon Atoms)} × 100

(Method for calculating degree of biomass (%) of polyisocyanate (1))

The degree of biomass of the polyisocyanate (a-II) was calculated on a carbon basis according to the degree of biomass of the 1,5-pentamethylene diisocyanate.

(Degree of biomass (%) of polyisocyanate (1))={Number of carbon atoms in 1,5-pentamethylene diisocyanate)x(Degree of biomass (%) of 1,5-pentamethylene diisocyanate)}/{(Number of carbon atoms in 1,5-pentamethylene diisocyanate)+(Number of carbon atoms in a compound added during the reaction)}

(Method for calculating degree of biomass (%) of episulfide compound (2))

The carbon-based degree of biomass of epichlorohydrin which was a plant-derived material was considered as 100%, and the degree of biomass of the synthesized episulfide compound was calculated in terms of carbon on the basis of the above-described degree of biomass.

Degree of biomass (%) of the episulfide compound={(Number of carbon atoms derived from epichlorohydrin in the episulfide compound)x(Degree of biomass (%) of epichlorohydrin)}/{(Number of carbon atoms in the episulfide compound)}

(Method for calculating degree of biomass (%) of polythiol compound (3))

The degree of biomass of the synthesized polythiol was calculated on a carbon basis by using epichlorohydrin which was obtained from a plant-derived material and had a degree of biomass of 100% on a carbon basis.

Degree of biomass of polythiol = {(Number of epichlorohydrin-derived carbon atoms in molecules of polythiol) × (Degree of biomass (%) of epichlorohydrin)} / {(Number of carbon atoms in molecules of polythiol)}

(Method for calculating degree of biomass (%) of resin for optical material)

The degree ofbiomass of the resin for an optical material was calculated on a carbon basis according to the degree of biomass of the polyisocyanate (1), the episulfide compound (2), and the polythiol compound (3).

(Degree of biomass (%) of the resin for an optical material)={(Number of carbon atoms in the polyisocyanate (1) used)x(Degree of biomass (%) of the polyisocyanate (1))+(Number of carbon atoms in the episulfide compound (2) used)x(Degree of biomass (%) of the episulfide compound (2))+(Number of carbon atoms in the polythiol compound (3) used)x(Degree of biomass (%) of the polythiol compound (3))}/{(Number of carbon atoms in the polyisocyanate (1) used)+(Number of all carbon atoms in other polyisocyanates)+(Number of carbon atoms in the episulfide compound (2) used)+(Number of carbon atoms in the polythiol compound (3) used)}

(Method for evaluating performance of lens)

The lens obtained by means of polymerization was evaluated by carrying out a performance test. The performance test was carried out in terms of the refractive index, the Abbe number, the heat resistance, and the dyeing properties, and these were evaluated using the following test methods.

- Refractive index (ne) and Abbe number (νe): The refractive index and the Abbe number were measured at 20°C using a Pulfrich refractometer KPR-30 manufactured by Shimadzu Corporation.
- Heat resistance: The glass transition temperature (Tg) in a TMA penetration method (with a load of 50 g and a pin tip diameter of 0.5 mmϕ) was set as heat resistance by using TMA-60 manufactured by Shimadzu Corporation.
- Dyeing properties: "FSP Red E-A" (manufactured by Futaba Corporation) (1.0 g), "FSP Yellow P-E" (manufactured by Futaba Corporation) (1.0 g), "FSP Blue AUL-S" (manufactured by Futaba Corporation) (2.0 g), "NICCA SUNSOLT #7000" (manufactured by Nicca Chemical Co., Ltd.) (4.0 g), and "DK-CN" (manufactured by Daiwa Chemical Industries Co., Ltd.) (4.0 g) were added to pure water (2, 986 g), thereby preparing a dye dispersed liquid. This liquid was heated to 90 °C, and then a 9 mm-thick molding piece was dyed by being immersed therein for five minutes at 90°C. The transmittance (%T) of the dyed molding piece at a wavelength of 565 nm was measured. Molding pieces

having a transmittance of lower than or equal to 40% after dyeing were evaluated as "having favorable dyeing properties" and molding pieces having a transmittance of higher than or equal to 60% were evaluated as "having poor dyeing properties".

[Reference Preparation Example]

(Synthesis of 1,5-pentamethylene diisocyanate using plant-derived material)

Preparation Example 1 (preparation of bacteria-disintegrated liquid)

(Cloning of lysine decarboxylase gene (cadA))

A genome DNA prepared from an Escherichia coli W3110 strain (ATCC27325) according to an ordinary method was used as a mold for PCR.

Oligonucleotides (synthesized by Invitrogen in trust) having base sequences shown in sequence numbers 1 and 2 which were designed on the basis of the base sequence of lysine decarboxylase gene (cadA) (GenBank Accession No. AP009048) were used as primers for PCR. These primers respectively have restriction enzyme recognition sequences of KpnI and XbaI near a 5' terminal.

A reaction cycle made up of denaturation at 94°C for 30 seconds, annealing at 55°C for 30 seconds, and an elongation reaction at 68°C for two minutes was carried out 30 times using a PCR reaction liquid (25 $\mu$L) including the genome DNA (1 ng/$\mu$L) and the respective primers (0.5 pmol/$\mu$L), thereby performing PCR.

The PCR reaction product and plasmid pUC18 (manufactured by Takara Shuzo Co . , Ltd.) were digested using KpnI and XbaI, were coupled together using LIGATION HIGH (manufactured by Toyobo Co., Ltd.), and the genetic transformation of Eschrichia coli DH5$\alpha$ (manufactured by Toyobo Co., Ltd.) was carried out using the obtained recombined plasmid. The genetic-transformed body was cultured in an LB agar medium including ampicillin (Am) (100 $\mu$g/mL) and X-Gal (5-bromo-4-chloro-3-indolyl-$\beta$-D-galactoside), thereby obtaining a genetic-transformed body which was resistant to Am and was formed of white colonies. Plasmids were extracted from the genetic-transformed body obtained in the above-described manner.

According to an ordinary method for determining base sequences, it was confirmed that the base sequence of a DNA fragment introduced into the plasmid was the base sequence shown in the sequence number 3.

A plasmid having a DNA that coded the obtained lysine decarboxylase was named as pCADA. Bacillus coli that had been genetic-transformed using pCADA was cultured, whereby lysine decarboxylase having the amino acid sequence shown in the sequence number 4 could be produced.

(Production of genetic-transformed body)

An Escherichia coli W3110 strain was genetic-transformed according to an ordinary method using pCADA, and the obtained genetic-transformed body was named as W/pCADA.

This genetic-transformed body was inoculated to an LB agar medium (500 ml) including Am (100$\mu$g/mL) in a three-neck flask equipped with a baffle and was shaken and cultured until OD (660 nm) reached 0.5 at 30°C, then, isopropyl-$\beta$-thiogalactopyranoside(IPTG) was added thereto so as to reach 0.1 mmol/L, and furthermore, the mixture was shaken and cultured for 14 hours. The culture solution was centrifugally separated at 8,000 rpm for 20 minutes, thereby obtaining bacteria.

These bacteria were suspended in a sodium phosphate buffer solution (pH 6.0) (20 mmol/L), and then ultrasonic disintegration was carried out, thereby preparing a bacteria-disintegrated liquid.

Preparation Example 2 (preparation of aqueous solution of 1,5-pentamethylene diamine)

To a flask, a substrate solution (120 parts by weight) prepared by adding L-lysine monohydrochloride (manufactured by Wako Pure Chemical Industries, Ltd.) so that the final concentration reached 45% by weight and pyridoxal phosphate (manufactured by Wako Pure Chemical Industries, Ltd.) so that the final concentration reached 0.15 mmol/L was added. Next, the W/pCADA bacteria-disintegrated liquid (finishing dried bacteria-equivalent weight: 0.3 g) was added thereto, and a reaction was initiated. The reaction conditions were set to 37°C and 200 rpm. The pH of a reaction liquid was adjusted to 6 using hydrochloric acid (6 mol/L) . After 24 hours, the reaction yield of 1,5-pentamethylene diamine reached 99%. The pH of the reaction liquid after 24 hours from the reaction was adjusted to 2 using hydrochloric acid (6 mol/L), activated carbon (manufactured by Mitsukura Chemical Co., Ltd., powdered activated carbon PM-SX) (0.6 parts by weight) was added thereto, and the components were stirred at 25°C for one hour, and then filtration was carried out using filter paper (5C manufactured by ADVANTEC Co., Ltd.). Next, the pH of this filtrate was adjusted to

12 using sodium hydroxide, thereby obtaining an aqueous solution of 1,5-pentamethylene diamine (17.0% by weight aqueous solution).

Preparation Example 3 (preparation of 1,5-pentamethylene diamine)

An aqueous solution of 1,5-pentamethylene diamine (100 parts by weight) and n-butanol (100 parts by weight) were prepared at 23°C in a separating funnel, were mixed together for 10 minutes, and then were left to stand for 30 minutes. The lower layer which was a water layer was removed, and then the upper layer which was an organic layer (n-butanol including 1,5-pentamethylene diamine) was removed. The extraction rate was measured and was found to be 91.6%. Next, the extraction liquid (80 parts by weight) of the organic layer was prepared in a four-neck flask equipped with a thermometer, a distillation tower, a cooling tube, and a nitrogen introduction tube, the oil bath temperature was set to 120°C, and n-butanol was distilled away at a reduced pressure of 10 kPa. Next, the oil bath temperature was set to 140 °C, and 1, 5-pentamethylene diamine was distilled away at a reduced pressure of 10 kPa, thereby obtaining 1, 5-pentamethylene diamine having a purity of 99.9% by weight.

Preparation Example 4 (manufacturing of 1,5-pentamethylene diisocyanate)

o-Dichlorobenzene (2,000 parts by weight) was prepared in a pressurization reactor equipped with a jacket provided with an electromagnetic induction stirrer, an automatic pressure adjustment valve, a thermometer, a nitrogen introduction line, a phosgene introduction line, condenser, and a raw material-feeding pump. Next, phosgene (2,300 parts by weight) was added from the phosgene introduction line, and stirring was initiated. Cooling water was made to flow through the gasket in the reactor, and the inside temperature was held at approximately 10°C. A solution obtained by dissolving o-dichlorobenzene (2,600 parts by weight) in 1,5-pentamethylene diamine (400 parts by weight) obtained in Preparation Example 1 was fed thereto using a feeding pump over 60 minutes, and cold phosgenation was initiated at lower than or equal to 30°C at normal pressure. After the end of the feeding, a light brown and white slurry-form liquid was formed in the pressurization reactor.

Next, the liquid in the reactor was pressurized to 0.25 MPa while being slowly heated to 160°C, and furthermore, hot phosgenation was carried out at a pressure of 0.25 MPa and a reaction temperature of 160°C for 90 minutes. Meanwhile, in the middle of the hot phosgenation, phosgene (1,100 parts by weight) was further added thereto. In the process of hot phosgenation, the liquid in the pressurization reactor turned into a light brown clear solution. After the end of the hot phosgenation, nitrogen gas was made to flow through at 100 L/hour at 100°C to 140°C and was degassed.

Next, o-dichlorobenzene was distilled away at reduced pressure, and then, similarly, 1,5-pentamethylene diisocyanate was distilled under reduced pressure, thereby obtaining 1,5-pentamethylene diisocyanate (558 parts by weight) having a purity of 98.7%.

Next, 1,5-pentamethylene diisocyanate (558 parts by mass) and tris(tridecyl)phosphate (manufactured by Johoku Chemical Co., Ltd, trade name: JP-333E) (0.02 parts by weight of 100 parts by weight of 1,5-pentamethylene diisocyanate) were loaded into a four-neck flask equipped with a stirrer, a thermometer, a reflux tube, and a nitrogen introduction tube and were heated at normal pressure at 210°C for two hours while introducing nitrogen into the flask, thereby obtaining 1,5-pentamethylene diisocyanate (553 parts by weight) having a purity of 98.3%. The yield of 1,5-pentamethylene diisocyanate in the thermal treatment was 99.6%.

Next, the heated 1,5-pentamethylene diisocyanate was loaded into a glass flask and was further purified while being refluxed under conditions of 127°C to 132°C and 2.7 KPa using a purification device equipped with a distillation tube loaded with four elements of the filled substance (manufactured by Sumitomo Heavy Industries, Ltd., trade name: Sumitomo/Sulzer Laboratory Packing EX-type), a distillation tower equipped with a reflux ratio adjustment timer (manufactured by Sibata Scientific Technology Ltd., trade name: distillation head K-type), and a cooler, thereby obtaining 1,5-pentamethylene diisocyanate having a purity of 99.9% by weight. The degree of biomass of this 1,5-pentamethylene diisocyanate was measured using the following method.

(Method for measuring degree of biomass (%) of 1,5-pentamethylene diisocyanate)

The degree of biomass (%) of 1,5-pentamethylene diisocyanate was calculated by measuring the basic value difference in the content of radioactive carbon (C14) from $CO_2$ generated by combusting a sample by means of accelerator mass spectrometry (AMS) according to the test method of American Society for Testing and Materials (ASTM) D6866 (Standard Test Method for Determining the Biobased Content of Natural Range Materials Using Radiocarbon and Isotope Ratio Mass Spectrometry Analysis).

1,5-Pentamethylene diisocyanate was made into methyl carbamate (urethane) using methanol, and the degree of biomass of the methyl carbamate substance was obtained by means of measurement according to the test method of ASTM D6866. As a result, the degree of biomass of the methyl carbamate body was 55.5%. The degree of biomass

of 1,5-pentamethylene diisocyanate was obtained from the above-described value using the following equation and was found to be 71%.

Degree of biomass (%) of 1,5-pentamethylene diisocyanate=(Degree of biomass (%) of the methyl carbamate body)x(Number of carbon atoms in the methyl carbamate)/(Number of carbon atoms in the 1,5-pentamethylene diisocyanate)

[Synthesis Example 1]

(Synthesis of modified 1,5-pentamethylene diisocyanate (preparation of polyisocyanate (1)))

1,5-Pentamethylene diisocyanate obtained in Preparation Example 4 (hereinafter, in some cases, abbreviated as PDI) (500 parts), isobutyl alcohol (hereinafter, in some cases, abbreviated as IBA) (1 part), 2,6-di(tert-butyl)-4-methyl phenol (hereinafter, in some cases, abbreviated as BHT) (0.3 parts), and tris(tridecyl)phosphite (manufactured by Johoku chemical Co., Ltd., trade name: JP-333E) (0.3 parts) were loaded into a four-neck flask equipped with a stirrer, a thermometer, a reflux tube, and a nitrogen introduction tube and were reacted together at 80°C for two hours. Next, N-(2-hydroxypropyl)-N,N,N-trimethylammonium-2-ethylhexanoate (manufactured by Air Products Japan, Inc. trade name: DABCO(R)TMR) (0.05 parts) were added thereto as a trimerization catalyst. After a 50-minute, o-toluenesulphoneamide (hereinafter, in some cases, abbreviated as OTS) (0.12 parts) was added thereto. The obtained reaction liquid was distilled at a degree of vacuum of 0.09 kPa and a temperature of 150 °C by being passed through a thin film distillation device, thereby obtaining unreacted pentamethylene diisocyanate (401 parts). Furthermore, o-toluenesulphoneamide (0.02 parts) was added to the obtained composition (100 parts), thereby obtaining polyisocyanate (1) (100 parts) which is a composition including a modified 1,5-pentamethylene diisocyanate. The amount derived from 1,5-pentamethylene diisocyanate constituting this modified product was 98% by weight.

The polyisocyanate (1) has a concentration of unreacted 1,5-pentamethylene diisocyanate of lower than 1% by weight, a concentration of mononuclear isocyanurate of 65% by weight, a number-average molecular weight of 554.7, a concentration of isocyanate groups of 25%, the average number of functional groups of 3.3, and a degree of biomass of 71%.

The degree of biomass of the polyisocyanate (1) is calculated using the following equation in terms of carbon on the basis of the degree of biomass of 1,5-pentamethylene diisocyanate of 71%. DABCO (R) TMR which was the catalyst used to form an isocyanurate was a mixture of N-(2-hydroxypropyl)-N,N,N-trimethyl ammonium-2-ethyl hexanoate (33.8% by weight), 2-ethylhexanoic acid (41.2% by weight), and ethylene glycol (25% by weight).

Ddegree of biomass of the polyisocyanate (1)={500 (parts by weight of PDI)/154.2 (the molecular weight of PDI)x7 (the total number of carbon atoms in one PDI molecule)x71 (degree of biomass (%) of PDI)}/{500 (parts by weight of PDI)/154.2 (the molecular weight of PDI)x7 (the total number of carbon atoms in one PDI molecule)+1 (part by weight of IBA)/74.1 (the molecular weight of IBA)x4 (the number of carbon atoms in one IBA molecule)+0.3 (parts by weight of BHT)/220.4 (the molecular weight of BHT)x15 (the number of carbon atoms in one BHT molecule)+0.3 (parts by weight of JP-333E)/628 (the molecular weight of JP-333E)x39 (the number of carbon atoms in one JP-333E molecule)+0.02 (parts by mass of N-(2-hydroxypropyl)-N,N,N-trimethylammonium-2-ethyl hexanoate)/118.2 (the molecular weight of N-(2-hydroxypropyl)-N,N,N-trimethylammonium-2-ethyl hexanoate)x6 (the number of carbon atoms in one N-(2-hydroxypropyl)-N,N,N-trimethylammonium-2-ethylhexanoate molecule)+0.02 (parts by weight of 2-ethylhexanoic acid)/144.2 (the molecular weight of 2-ethylhexanoic acid)x8 (the number of carbon atoms in one 2-ethylhexanoic acid molecule)+0.01 (parts by weight of ethylene glycol)/62.1 (the molecular weight of ethylene glycol)x2 (the number of carbon atoms in one ethylene glycol molecule)+0.12 (parts by mass of OTS)/171.2 (the molecular weight of OTS)x7 (the number of carbon atoms in one OTS molecule)}=71%

[Synthesis Example 2] (Synthesis of episulfide compound (2))

Epichlorohydrin manufactured from plant-derived glycerin (EPICHLOROHYDRIN(ECH) manufactured by Solvay) (190 g (2 mol)), methanol (500 ml), and calcium hydroxide (1.0 g) were prepared in a reaction flask equipped with a stirring rod, a thermometer, a gas sealing tube, and a condenser, the inside temperature was held in a range of 0°C to 5°C under stirring, hydrogen sulfide gas (75 g, 2.2 mol) was blown into the reaction system through the gas sealing tube over two hours, and the components were aged at 5°C for three hours.

The reaction liquid was filtered, methanol was desolvated, and then the residue was distilled away, thereby obtaining chloromercaptopropanol. The obtained chloromercaptopropanol, pure water (1,000 ml), and sodium hydrogen

carbonate (168 g, 2 mol) were prepared, solid iodine (254 g, 1 mol) was loaded in a split manner over one hour while holding the inside temperature in a range of 5°C to 10°C, and the components were aged at 10°C for 12 hours. The reaction liquid that had been aged was filtered, and the obtained white crystals were dried at reduced pressure.

The dried white crystals, methanol (250 ml), and toluene (500 ml) were again loaded into the reactor, 47 wt% caustic soda (240 g, 2.8 mol) was added dropwise for one hour while holding the inside temperature in a range of 3°C to 5°C, and the components were aged for 30 minutes. After the end of the reaction, toluene (100 ml) was added thereto, and the organic layer was washed with pure water three times. The filtrate obtained by dehydrating the obtained organic layer with anhydrous magnesium sulfate and filtering the organic layer was desolvated. After the desolvation, the residual was filtered, and bis(2,3-epoxypropyl) disulfide was obtained (yield: 92%).

Bis(2,3-epoxypropyl) disulfide) (100 g, 0.54 mol), thiourea (100 g, 1.3 mol), acetic acid (2 g), toluene (250 ml), and methanol (200 ml) were prepared in a reaction flask equipped with a stirrer, a thermometer, and a condenser, the inside temperature was held at 15°C, and the components were stirred for 16 hours. After the end of the reaction, toluene (150 ml) was added thereto, and the mixture was washed with saline solution, 1% sulfuric acid water, and, again, saline solution.

The filtrate obtained by dehydrating the obtained organic layer with anhydrous magnesium sulfate and filtering the organic layer was desolvated. Acetonitrile (600 ml) was added to and dissolved in the desolvated residue, and the supernatant solution was filtered.

The residue of the obtained filtrate after the desolvation was filtered, thereby obtaining a composition (77.5 g) (the purity-equivalent yield was 58%) of a thioepoxy compound containing 85 wt% of bis(2,3-epithiopropyl)disulfide. This composition (50 g) of a thioepoxy compound having a purity of 85 wt% was sorted by means of silica gel column chromatography, thereby obtaining an episulfide compound (2) (38 g).

The degree of biomass of the episulfide compound (2) is calculated using the following equation in terms of carbon on the basis of the degree of biomass of epichlorohydrin of 100%.

Degree of biomass of the episulfide compound (2)={(Number of carbon atoms derived from epichlorohydrin in the episulfide compound (2))x(Degree of biomass (%) of epichlorohydrin)}/{(Number of carbon atoms in the episulfide compound (2))}=100%

[Synthesis Example 3] (Synthesis of polythiol compound (3))

(Synthesis of polythiol including 4,8 and 4,7 and 5,7-dimercaptomethyl-1,11-dimercapto-3,6,9-trithiaundecane as main component)

2-Mercaptoethanol (51.2 parts by weight), degassed water (dissolved oxygen level: 2 ppm) (26. 5 parts by weight), and an aqueous solution of 49% by weight of sodium hydroxide (0.16 parts by weight) were loaded into a reactor. Epichlorohydrin manufactured from plant-derived glycerin (EPICHLOROHYDRIN(ECH) manufactured by Solvay) (61.99 parts by weight) was added dropwise and loaded over 6.5 hours at 9°C to 11°C and, subsequently, was stirred for 60 minutes.

Next, an aqueous solution of 17.3% soda sulfide (150.0 parts by weight) was added dropwise and loaded over 5.5 hours at 7°C to 37°C and, subsequently, was stirred for 120 minutes.

In addition, 35.5% hydrochloric acid (279.0 parts by weight) was loaded thereinto, then, thiourea having a purity of 99.90% (125.8 parts by weight) was loaded thereinto, and the components were stirred for three hours under a reflux at 110°C, thereby causing a thiuronium chlorination reaction. After the cooling of the mixture to 45°C, toluene (214.0 parts by weight) was added thereto, the mixture was cooled to 26°C, an aqueous solution of 25% by weight of ammonia (206.2 parts by weight) was loaded thereinto at 26°C to 50°C over 30 minutes, and the components were stirred at 50°C to 65°C for one hour so as to cause a hydrolysis reaction, thereby obtaining a toluene solution of the target polythiol compound. 36% Hydrochloric acid (59.4 parts by weight) was added to the toluene solution, and the mixture was washed with an acid at 34°C to 39°C for 30 minutes twice. Degassed water (dissolved oxygen level: 2 ppm) (118.7 parts by weight) was added thereto and was washed at 35°C to 45°C for 30 minutes five times. After the toluene and a small amount of moisture were removed under heating and depressurization, the mixture was filtered at reduced pressures using a 1.2 μm PTFE-type membrane filter, thereby obtaining a polythiol compound (115.9 parts by weight) including 4,8-dimercaptomethyl-1,11-dimercapto-3,6,9-trithiaundecane (Compound A), 4,7-dimercaptomethyl-1,11-dimercapto-3,6,9-trithiaundecane (Compound B), and 5,7-dimercaptomethyl-1,11-dimercapto-3,6,9-trithiaundecane (Compound C)

as main components (an isomer mixture of Compounds A/B/C=85/5/10 (molar ratio)).

The degree of biomass of the polythiol compound (3) is calculated using the following equation in terms of carbon on the basis of the degree of biomass of epichlorohydrin of 100%.

Degree of biomass of the polythiol compound (3)={(Number of carbon atoms derived from epichlorohydrin in the polythiol compound (3))x(Degree of biomass (%) of epichlorohydrin)}/{(Number of carbon atoms in the polythiol compound (3))}=60%

[Example 1]

Bis(2,3-epithiopropyl) disulfide (the episulfide compound (2); 6.0 g) and (the polythiol compound (3); 4.4 g) including 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane as main components were sequentially added to a mixture of tetrabutylammonium bromide (17.4 mg) and acidic phosphoric acid ester (ZelecUN; 8.7 mg) and were stirred at room temperature for 15 minutes. After confirming that the solid substance was dissolved, the polyisocyanate (1) (7 g) was sequentially added thereto and was stirred and degassed at reduced pressure for 30 minutes, thereby preparing a liquid mixture. The liquid mixture was filtered using a 1 μm PTFE filter and then was injected into a glass mold for 2C plano having a central thickness of 2 mm and a diameter of 80 mm. This glass mold was heated to 30°C to 120°C for 15 hours and then was cooled to 25°C for one hour, and the liquid mixture was removed from the glass mold, thereby obtaining a lens. The obtained lens was further annealed at 120°C for three hours. The obtained lens had a refractive index (ne) of 1.64, an Abbe number of 37, a glass transition temperature (Tg) of 78°C, and a degree of biomass of 78%. The dyeing properties were also favorable.

(3))/366.7 (the molecular weight of the polythiol compound (3))x10 (the number of carbon atoms in one polythiol compound (3) molecule)x60 (degree of biomass % of the polythiol compound (3))}]/[6 (parts by weight of the episulfide compound (2))/210.4 (the molecular weight of the episulfide compound (2)x6 (the number of carbon atoms in one episulfide compound (2) molecule))+{7 (parts by weight of the polyisocyanate (1))/554.7 (the number-average molecular weight of the polyisocyanate (1))x23 (the number of carbon atoms in an average molecule of the polyisocyanate (1))+{4.4 (parts by weight of the polythiol compound (3))/366.7 (the molecular weight of the polythiol compound (3))x10 (the number of carbon atoms in one polythiol compound (3) molecule)}]=78%

[Example 2]

Bis (2, 3-epithiopropyl) disulfide (the episulfide compound (2); 6.0 g) and (the polythiol compound (3); 4.4 g) including 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane as main components were sequentially added to a mixture of tetrabutylammonium bromide (16.0 mg) and acidic phosphoric acid ester (ZelecUN; 8.1 mg) and were stirred at room temperature for 15 minutes. After confirming that the solid substance was dissolved, the polyisocyanate (1) (2.70 g) and m-xylylene diisocyanate (3.0 g) were sequentially added thereto and was stirred and degassed at reduced pressure for 30 minutes, thereby preparing a liquid mixture. The liquid mixture was filtered using a 1 μm PTFE filter and then was injected into a glass mold for 2C plano having a central thickness of 2 mm and a diameter of 80 mm. This glass mold was heated to 30 °C to 120°C for 15 hours and then was cooled to 25°C for one hour, and the liquid mixture was removed from the glass mold, thereby obtaining a lens. The obtained lens was further annealed at 120°C for three hours. The obtained lens had a refractive index (ne) of 1.67, an Abbe number of 32, and a degree of biomass of 57%. The dyeing properties were also favorable.

Degree of biomass (%) of the resin=[{6 (parts by weight of the episulfide compound (2))/210.4 (the molecular weight of the episulfide compound (2))x6 (the number of carbon atoms in one episulfide compound (2) molecule)x100 (degree of biomass % of the episulfide compound (2))}+{2.7 (parts by weight of the polyisocyanate (1))/554.7 (the number-average molecular weight of the polyisocyanate (1))x23 (the number of carbon atoms in an average molecule of the polyisocyanate (1))x71 (degree of biomass % of the polyisocyanate (1))}+{4.4 (parts by weight of the polythiol compound (3))/366.7 (the molecular weight of the polythiol compound (3))x10 (the number of carbon atoms in one polythiol compound (3) molecule)x60 (degree of biomass % of the polythiol compound (3))}]/[6 (parts by weight of the episulfide compound (2))/210.4 (the molecular weight of the episulfide compound (2)x6 (the number of carbon atoms in one episulfide compound (2) molecule))+{2.7 (parts by weight of the polyisocyanate (1))/554.7 (the number-average molecular weight of the polyisocyanate (1))x23 (the number of carbon atoms in an average molecule of the polyisocyanate (1))+{4.4 (parts by weight of the

As described above, in Examples 1 and 2, it was possible to obtain sulfide-based resins for optical materials which had a degree of biomass of higher than or equal to 25% and satisfied properties such as transparency, refractive index, and heat resistance from a polymerizable composition including an episulfide compound obtained using a plant-derived material. In addition, in Example 1, both the polythiol compound and the aliphatic polyisocyanate compound were combined only with a compound manufactured from plant-derived materials, and it was possible to obtain a resin for an optical material which had a high degree of biomass of higher than or equal to 70% and satisfied properties such as transparency, refractive index, and heat resistance. In Example 2, m-xylylene diisocyanate was added thereto, and thus it was possible to obtain a resin for an optical material which had a high degree of biomass of higher than or equal to 25%, satisfied transparency, refractive index, and the like, and, simultaneously, had excellent dyeing properties.

The present application claims priority on the basis of Japanese Patent Application No. 2014-047890 filed on March 11, 2014 and Japanese Patent Application No. 2014-185995 filed on September 12, 2014, the contents of which are incorporated herein.

## Claims

**1.** A polymerizable composition for an optical material including:

polyisocyanate (a) including aliphatic polyisocyanate (a1) and a modified aliphatic polyisocyanate (a2);
an episulfide (b); and
a polythiol (c).

**2.** The polymerizable composition for an optical material according to claim 1,

wherein the modified aliphatic polyisocyanate (a2) is an isocyanurate of the aliphatic polyisocyanate.

**3.** The polymerizable composition for an optical material according to claim 1 or 2,
wherein the aliphatic polyisocyanate (a1) and/or the aliphatic polyisocyanate in the modified aliphatic polyisocyanate (a2) are obtained from plant-derived materials.

**4.** The polymerizable composition for an optical material according to any one of claims 1 to 3,
wherein the episulfide (b) includes an episulfide represented by Formula (1):

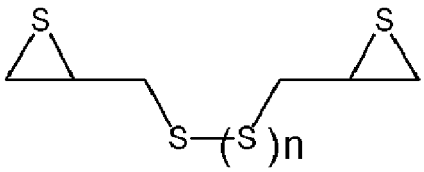

(1)

wherein in the formula (1), n represents 0 or 1.

**5.** The polymerizable composition for an optical material according to any one of claims 1 to 4,
wherein the episulfide (b) includes an episulfide obtained from plant-derived materials.

**6.** The polymerizable composition for an optical material according to any one of claims 1 to 5,
wherein the aliphatic polyisocyanate (a1) and/or the aliphatic polyisocyanate in the modified aliphatic polyisocyanate (a2) is at least one selected from 1,5-pentamethylene diisocyanate, 1,6-hexamethylene diisocyanate, 1,7-heptamethylene diisocyanate, lysine diisocyanate, lysine triisocyanate, dimer acid diisocyanate, octamethylene diisocyanate, and decamethylene diisocyanate.

**7.** The polymerizable composition for an optical material according to any one of claims 1 to 6,
wherein the polythiol (c) is at least one selected from 4-mercaptomethyl-1,8-dimercapto-3,6-dithiaoctane, 4,8 or 4,7 or 5,7-dimercaptomethyl-1,11-dimercapto-3,6,9-trithiaundecane, pentaerythritol tetrakis mercaptoacetate, pentaerythritol tetrakis mercaptopropionate, 2,5-bis(mercaptomethyl)-1,4-dithiane, bis(mercaptoethyl)sulfide, 1,1,3,3-tetrakis(mercaptomethylthio)propane, 4,6-bis(mercaptomethylthio)-1,3-dithiane, 2-(2,2-bis(mercaptomethylthio) ethyl)-1,3-dithietane, 1,1,2,2-tetrakis(mercaptomethylthio) ethane, 3-mercaptomethyl-1,5-dimercapto-2,4-dithiapentane, tris(mercaptomethylthio) methane, and ethylene glycol bis(3-mercaptopropionate).

**8.** The polymerizable composition for an optical material according to any one of claims 1 to 7,
wherein the polythiol (c) is a polythiol obtained from plant-derived materials, and is selected from 4-mercaptomethyl-1,8-dimercapto-3,6-dithiaoctane and 4,8 or 4,7 or 5,7-dimercaptomethyl-1,11-dimercapto-3,6,9-trithiaundecane.

**9.** A molding comprising:

a resin obtained by polymerizing and curing the polymerizable composition for an optical material according to any one of claims 1 to 8.

**10.** The molding according to claim 9,
wherein the resin has a degree of biomass of greater than or equal to 25%.

**11.** The molding according to claim 9 or 10,
wherein the molding has a refractive index of higher than or equal to 1.60.

**12.** The molding according to any one of claims 9 to 11,
wherein the molding has a glass transition temperature (Tg) of higher than or equal to 60°C.

**13.** An optical material formed of the molding according to any one of claims 9 to 12.

**14.** A plastic spectacle lens formed of the molding according to any one of claims 9 to 12.

**15.** A plastic polarizing lens, comprising:

a polarizing film; and
a layer formed of the molding according to any one of claims 9 to 12, wherein the layer is laminated on at least one surface of the polarizing film.

**16.** A method for manufacturing a plastic spectacle lens, comprising:

a step of injecting the polymerizable composition for an optical material according to any one of claims 1 to 8 into a mold for lens casting; and
a step of polymerizing and curing the polymerizable composition for an optical material.

**17.** A method for manufacturing a plastic polarizing lens, comprising:

a step of fixing a polarizing film into a lens casting mold in a state in which at least one surface of the polarizing film is separated from a mold;
a step of injecting the polymerizable composition for an optical material according to any one of claims 1 to 8 into a gap between the polarizing film and the mold; and
a step of laminating a layer formed of a polythiourethane resin on at least one surface of the polarizing film by polymerization and curing the polymerizable composition for an optical material.

**INTERNATIONAL SEARCH REPORT**

International application No.
PCT/JP2015/057165

A. CLASSIFICATION OF SUBJECT MATTER
*C08G18/38*(2006.01)i, *C08G18/79*(2006.01)i, *G02B1/04*(2006.01)i, *G02C7/02*(2006.01)i, *G02C7/12*(2006.01)i

According to International Patent Classification (IPC) or to both national classification and IPC

B. FIELDS SEARCHED

Minimum documentation searched (classification system followed by classification symbols)
C08G18/00-18/87, C08G75/00-75/32, G02B1/00-1/18, G02C7/00-7/16

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | JP 2012-167199 A (Mitsubishi Gas Chemical Co., Inc.), 06 September 2012 (06.09.2012), claims (particularly, claim 3); chemical formula 1; paragraphs [0024], [0027] & CN 102634024 A | 1-17 |
| Y | JP 2013-209628 A (Mitsubishi Gas Chemical Co., Inc.), 10 October 2013 (10.10.2013), claims (particularly, claim 3); chemical formula 3; paragraphs [0012], [0014] & KR 10-2013-0100714 A & CN 103289094 A | 1-17 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 June 2015 (03.06.15) | 16 June 2015 (16.06.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No. PCT/JP2015/057165

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2004-256746 A (Mitsui Chemicals, Inc.), 16 September 2004 (16.09.2004), claims; paragraphs [0023] to [0038] (Family: none) | 1-17 |
| Y | JP 2002-122701 A (Mitsubishi Gas Chemical Co., Inc.), 26 April 2002 (26.04.2002), claims; paragraphs [0008] to [0017] & US 2004/0122201 A1 & WO 2002/033448 A1 & EP 1326095 A1 | 1-17 |
| Y | JP 2000-256435 A (Mitsui Chemicals, Inc.), 19 September 2000 (19.09.2000), claims; paragraphs [0014], [0022] to [0047] (Family: none) | 1-17 |
| Y | JP 2011-12141 A (Mitsui Chemicals, Inc.), 20 January 2011 (20.01.2011), claims; paragraphs [0009], [0020] (Family: none) | 1-17 |
| Y | WO 2004/108787 A1 (Hoya Corp.), 16 December 2004 (16.12.2004), claims; description, page 8, 7th to 2nd lines from the bottom; description, page 14, 10th to 5th lines from the bottom & US 2006/0149018 A1 & EP 1637553 A1 | 1-17 |
| Y | JP 2003-98301 A (Nippon Polyurethane Industry Co., Ltd.), 03 April 2003 (03.04.2003), claims; paragraphs [0005], [0011] (Family: none) | 1-17 |
| A | WO 2012/121291 A1 (Mitsui Chemicals, Inc.), 13 September 2012 (13.09.2012), claims & US 2013/0338330 A1 & EP 2684867 A1 | 1-17 |
| A | JP 2008-51851 A (Seiko Epson Corp.), 06 March 2008 (06.03.2008), claims (Family: none) | 1-17 |
| A | JP 2005-220162 A (Seiko Epson Corp.), 18 August 2005 (18.08.2005), claims (Family: none) | 1-17 |
| A | JP 2007-90574 A (Hoya Corp.), 12 April 2007 (12.04.2007), claims (Family: none) | 1-17 |

Form PCT/ISA/210 (continuation of second sheet) (July 2009)

**INTERNATIONAL SEARCH REPORT**

International application No.
PCT/JP2015/057165

C (Continuation). DOCUMENTS CONSIDERED TO BE RELEVANT

| Category* | Citation of document, with indication, where appropriate, of the relevant passages | Relevant to claim No. |
|---|---|---|
| A | JP 2001-131257 A (Mitsubishi Gas Chemical Co., Inc.), 15 May 2001 (15.05.2001), claims & US 6472495 B1 & EP 1099721 A1 | 1-17 |
| A | JP 2005-272785 A (Mitsui Chemicals, Inc.), 06 October 2005 (06.10.2005), claims (Family: none) | 1-17 |
| A | JP 2005-272778 A (Mitsui Chemicals, Inc.), 06 October 2005 (06.10.2005), claims (Family: none) | 1-17 |
| A | JP 2006-1982 A (Mitsui Chemicals, Inc.), 05 January 2006 (05.01.2006), claims (Family: none) | 1-17 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002194083 A
- JP 2000256435 A
- JP 2001163874 A
- WO 2013115212 A
- WO 2013157490 A
- JP 2013142073 A
- JP 2013541531 W
- JP 2010190919 A
- JP 2011225863 A
- JP 2014047890 A
- JP 2014185995 A